(19) Europäisches Patentamt / European Patent Office / Office européen des brevets

(11) **EP 3 209 792 B1**

(12) **FASCICULE DE BREVET EUROPEEN**

(45) Date de publication et mention de la délivrance du brevet:
**20.05.2020 Bulletin 2020/21**

(21) Numéro de dépôt: **15791711.3**

(22) Date de dépôt: **23.10.2015**

(51) Int Cl.:
*C12Q 1/24* (2006.01)  *C12N 13/00* (2006.01)
*G01N 1/40* (2006.01)  *G01N 15/10* (2006.01)
*G01N 15/14* (2006.01)

(86) Numéro de dépôt international:
**PCT/FR2015/052848**

(87) Numéro de publication internationale:
**WO 2016/062974 (28.04.2016 Gazette 2016/17)**

(54) **MÉTHODE DE TRAITEMENT D'ÉCHANTILLONS BIOLOGIQUES, NOTAMMENT D'ÉCHANTILLONS ALIMENTAIRES**

VERFAHREN ZUR BEHANDLUNG VON BIOLOGISCHEN PROBEN, INSBESONDERE VON LEBENSMITTELPROBEN

METHOD FOR TREATING BIOLOGICAL SAMPLES, ESPECIALLY FOOD SAMPLES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **24.10.2014 FR 1460258**

(43) Date de publication de la demande:
**30.08.2017 Bulletin 2017/35**

(73) Titulaire: **Biomérieux**
**69280 Marcy-L'Etoile (FR)**

(72) Inventeurs:
• **BROYER, Patrick**
**F-38500 Saint Cassien (FR)**
• **PATEL, Pradip**
**London SW17 7LT (GB)**
• **PAMME, Nicole**
**Beverley HU17 7JL (GB)**
• **RAYMOND, Jean-Claude**
**F-69690 Bessenay (FR)**

(74) Mandataire: **Le Mauff, Frédéric Francis Joseph**
**bioMérieux**
**376, chemin de l'Orme**
**69280 Marcy-l'Étoile (FR)**

(56) Documents cités:
**WO-A1-2014/046605     FR-A1- 2 985 520**

• **PER AUGUSTSSON ET AL: "Decomplexing biofluids using microchip based acoustophoresis", LAB ON A CHIP, vol. 9, no. 6, 1 janvier 2009 (2009-01-01), pages 810-818, XP055183122, ISSN: 1473-0197, DOI: 10.1039/B811027A**
• **ANDREAS LENSHOF ET AL: "Acoustofluidics 8: Applications of acoustophoresis in continuous flow microsystems", LAB ON A CHIP, vol. 12, no. 7, 7 mars 2012 (2012-03-07), pages 1210-1223, XP055207879, ISSN: 1473-0197, DOI: 10.1039/c2lc21256k**
• **ANDERS STÅHLBERG ET AL: "The workflow of single-cell expression profiling using quantitative real-time PCR", EXPERT REVIEW OF MOLECULAR DIAGNOSTICS, vol. 14, no. 3, 1 avril 2014 (2014-04-01), pages 323-331, XP055208042, ISSN: 1473-7159, DOI: 10.1586/14737159.2014.901154**
• **LENSHOF A ET AL: "Emerging Clinical Applications of Microchip-Based Acoustophoresis", JOURNAL OF LABORATORY AUTOMATION, vol. 16, no. 6, 1 décembre 2011 (2011-12-01), pages 443-449, XP028113041, ISSN: 2211-0682, DOI: 10.1016/J.JALA.2011.07.004 [extrait le 2011-07-06]**
• **JULIEN AUTEBERT ET AL: "Microfluidic: An innovative tool for efficient cell sorting", METHODS, vol. 57, no. 3, 1 juillet 2012 (2012-07-01), pages 297-307, XP055208151, ISSN: 1046-2023, DOI: 10.1016/j.ymeth.2012.07.002**

- **Mark D Tarn ET AL: "On-chip processing of particles and cells via multilaminar flow streams", Analytical and Bioanalytical Chemistry, 23 octobre 2013 (2013-10-23), pages 139-161, XP055208110, Berlin/Heidelberg DOI: 10.1007/s00216-013-7363-6 Extrait de l'Internet: URL:http://www.ncbi.nlm.nih.gov/pubmed/241 50283 [extrait le 2015-08-17]**
- **L. C. JELLEMA ET AL: "Charge-based particle separation in microfluidic devices using combined hydrodynamic and electrokinetic effects", LAB ON A CHIP, vol. 9, no. 13, 26 March 2009 (2009-03-26) , page 1914, XP055061744, ISSN: 1473-0197, DOI: 10.1039/b819054b**

Remarques:
Le dossier contient des informations techniques présentées postérieurement au dépôt de la demande et ne figurant pas dans le présent fascicule.

**Description**

La méthode selon l'invention et les dispositifs permettant la mise en œuvre de la méthode selon l'invention présentent un intérêt pour le traitement d'échantillons biologiques, notamment dans le domaine des tests sur des échantillons alimentaires.

Les intoxications d'origine alimentaire constituent depuis longtemps une menace pour la santé publique. Un total de 19 531 infections, 4.563 hospitalisations et 68 décès associés aux intoxications d'origine alimentaire, par exemple de *Salmonella, Escherichia Coli* O157:H7, *Listeria* ou *Campylobacter,* ont ainsi été signalés en 2012 par le réseau FoodNet. Des procédés classiques de recherche de la présence d'agents pathogènes dans les aliments comprennent :

- Un pré-enrichissement de l'échantillon afin de permettre la remise en phase de croissance de l'agent pathogène suspecté d'être contenu ainsi que de la flore intrinsèque présente naturellement dans l'échantillon ;
- Un enrichissement sélectif par culture pour inhiber la croissance de la flore intrinsèque, permettant d'obtenir un meilleur rapport de concentration entre l'agent pathogène et le « bruit de fond » ;
- Une culture sélective sur boîte de Petri pour permettre la croissance de l'agent pathogène spécifique, tout en limitant ou en inhibant la croissance de la flore intrinsèque.

Grâce à cette méthode, l'agent pathogène, s'il est présent, est détecté comme une colonie «typique» de couleur sur la boîte de Petri.

D'autres part, différentes techniques biochimiques, immunologiques, des tests sérologiques ou encore génétiques peuvent permettre une identification plus fine de l'agent pathogène.

Ce procédé classique est donc assez lourd en manipulation, et demande beaucoup de temps et de main-d'œuvre. A titre d'exemple, la détection et l'identification de *Salmonella* par ce procédé peut prendre de 3 à 5 jours.

De nombreux systèmes de détection rapide, y compris basés sur l'immunologie (par exemple l'appareil VIDAS® 3 de la demanderesse ou le dispositif décrit dans la publication de PerAugustsson et al. « Decomplexing Biofluids using microchip based acoustophoresis », LAB ON CHIP 2009, Vol 9, no 6, pages 801-818 ou le dispositif décrit dans la publication de L.C. JELLEMA et Al « Acoustofluidics 8 : Applications of acoustophoresis in continuous flow microsvstems » LAB ON CHIP 2012, vol. 12, no7, pages 1210-1223, ou des techniques de biologie moléculaire sont également disponibles et permettent de réduire le temps d'analyse de 24h à 48h, en fonction des types d' agents pathogènes et de la matrice de l'échantillon alimentaire.

En raison de la chaîne d'approvisionnement alimentaire mondiale et des pressions réglementaires visant à accroître la sécurité des aliments pour le consommateur, il existe un besoin accru de fournir un procédé plus rapide permettant de détecter et/ou d'identifier des agents pathogènes dans des matrices alimentaires. D'autre part, les instances règlementaires pour la sureté des aliments imposent la confirmation d'un résultat d'analyse par une méthode différente de celle ayant fourni le premier résultat.

Dans ce but, plusieurs approches basées sur la capture et la concentration de l'agent pathogène cible à partir d'échantillons complexes avant la détection ont ainsi été rapportées. Ces procédés utilisent notamment des protéines de bactériophages ou des particules immunomagnétiques recouvertes de micro ou nano-billes magnétiques. Ces techniques permettent une détection plus rapide et plus robuste de l'agent pathogène à partir de certaines matrices alimentaires en raison de la réduction des interférences d'arrière-plan (par exemple les inhibiteurs de PCR pour la détection moléculaire), ainsi que la concentration de l'agent pathogène. Cependant, ces techniques sont particulièrement limitées pour une analyse sur des matrices alimentaires comprenant des composants gras, ceux-ci pouvant entraver la récupération des billes de capture. D'autre part ces techniques présentent un coût relativement élevé, totalement incompatible avec une utilisation en routine.

Eu égard aux problématiques présentées ci-dessus, un objectif de la présente invention vise à mettre au point une méthode permettant :

- de proposer un procédé simplifié et universel permettant de traiter et d'analyser des échantillons alimentaires de différents types, y compris ceux comprenant un grand nombre de composants gras ou particulaires.
- de proposer un procédé garantissant la viabilité des particules, cellules ou molécules d'intérêt, qui peuvent être en outre analysées, notamment la viabilité des agents pathogènes tel que des bactéries.
- de proposer un procédé permettant une remise en phase de croissance plus rapide des agents pathogènes et de la flore intrinsèque présente dans l'échantillon biologique
- de séparer et/ou de concentrer des particules, cellules ou molécules d'intérêt a des débits élevés, notamment des débits supérieurs à 1 μL/min par canal de séparation pour traiter des volumes d'échantillons significatifs (plusieurs mL si nécessaire).
- de limiter l'obtention d'éléments non-spécifiques susceptibles de fausser le résultat de l'analyse in fine,
- de limiter au maximum les risques de contamination de l'échantillon et/ou de l'environnement et/ou du technicien de laboratoire,

- de simplifier le protocole de préparation d'échantillons alimentaires, afin de réduire le temps opérateur, le nombre de matériels et de consommables utilisés, les risques d'erreurs et améliorer la reproductibilité,
- de proposer des dispositifs et une méthode compatible avec l'ensemble des méthodes d'analyse connues (microbiologie, culture, virologie, bactériologie, immunoessais, métaséquençage, PCR...).
- de décomplexifier un échantillon alimentaire, éventuellement capturer, et d'analyser les particules d'intérêt susceptibles d'être présentes dans l'échantillon en flux continu,
- de décomplexifier l'échantillon alimentaire en séparant les particules non-spécifiques vers une des sorties d'un dispositif microfluidique.
- de proposer plusieurs dispositifs microfluidiques, supports et systèmes associés susceptibles de mettre en œuvre des méthodes de traitement d'échantillon biologiques par acoustophorèse, selon l'invention.

La présente invention concerne une méthode permettant la décomplexification d'échantillons biologiques, notamment alimentaires, permettant ainsi la capture et/ou l'analyse de microorganismes contenus ou susceptibles d'être contenus dans l'échantillon biologique.

Afin d'atteindre cet objectif et de palier aux inconvénients des méthodes précitées, l'invention concerne une méthode de traitement d'un échantillon biologique telle que définie par la revendication 1.

Par particules non-spécifiques, on entend des particules pouvant gêner ou limiter la capture et/ou la détection de microorganismes dans l'échantillon biologique. De telles particules peuvent être, à titre d'exemple, des débris alimentaires, des composés de fibres musculaires, des amas de graisses, de la pulpe, des micelles, coagulum, etc.

A la suite de cette méthode, au moins un des orifices de sortie du dispositif présente un échantillon décomplexifié, c'est à dire que sa concentration en particules non-spécifiques, tel que des débris alimentaires est réduite. Cette étape de décomplexification est donc analogue a une étape de purification dans la mesure où elle facilite la détection et l'analyse ultérieure des microorganismes présents dans l'échantillon. En effet, la majorité des particules non-spécifiques est séparée par l'étape de séparation par acoustophorèse, celles-ci présentant un volume et/ou une densité plus importante et/ou une compressibilité moins importante que les microorganismes, elles sont donc plus sujettes à la pression de radiation acoustique et se retrouvent concentrés dans un des orifices de sortie. Au contraire, la flore intrinsèque et éventuellement les espèce(s) d'intérêt subissent moins l'effet de l'onde acoustique de par leur taille leur densité et leur compressibilité. Celles-ci vont donc être réparties majoritairement par la circulation du fluide les contenant lors de leur introduction dans le dispositif. En fonction de la géométrie du dispositif et l'orientation de l'onde acoustique dans le canal de séparation, les particules non-spécifiques peuvent notamment être séparées en direction d'un orifice de sortie dont le canal d'acheminement présente un axe semblable et dans le prolongement de celui du canal de séparation. Cette séparation en flux continu vers un orifice de sortie « central », dont le canal présente un axe semblable à celui du canal de séparation peut être réalisée en appliquant une onde acoustique présentant une nœud de pression sensiblement centré sur l'axe longitudinal du canal de séparation. La répartition de la flore intrinsèque et éventuellement des espèce(s) d'intérêt, peut, en fonction de leur volume, de leur densité et de leur compressibilité, être équitable entre les orifices de sortie ou concentrée dans au moins un orifice de sortie, différent de celui où se concentre les particules non-spécifiques.

A la suite de cette méthode, au moins un des orifices de sortie du dispositif, différent de celui comprenant l'échantillon enrichi et décomplexifié, présente un échantillon concentré, c'est à dire que sa concentration en particules non-spécifiques est augmentée.

Des solutions tampons pouvant être utilisées sont par exemple et de façon non-limitative :

- Des tampons neutres, par exemple de l'eau, une solution de NaCl à 0,85%, un tampon phosphate salin (Phosphate Buffer Saline ou PBS en langue anglaise).
- Des tampons de remise en croissance non-spécifiques (génériques) de microorganismes. A titre d'exemple ces tampons peuvent être de type BHI (« brain heart infusion broth» en langue anglaise), TSB (bouillon Trypticase Soja), BPW (eau peptonée).
- Des tampons de remise en croissance spécifiques de microorganismes. A titre d'exemple ces tampons peuvent être de type : Fraser 1/2, bouillon LX (Listeria Xpress broth ; Ref. 42626 bioMérieux, France), bouillon LMX (Listeria Monocytogenes Xpress broth ; bioMérieux, France ou bouillon SX (Salmonella Xpress broth ; Ref. 42118 bioMérieux, France)
- Des tampons de densité soit inférieure soit supérieure à la densité de l'échantillon biologique, la densité de l'échantillon est supposée proche de 1. A titre d'exemple ces tampons peuvent comprendre : de la silice colloïdale (silanisée), du Iohexol (Nycodenz®), du Iodixanol (Opti-Prep^tm), du Ficoll 400, du Dextran 70, du Dextran 200, du Dextran 500, du sucrose, du chlorure de césium, des fluides de perfluorocarbone, des huiles minérales, des huiles de silicone, des huiles pour objectifs de microscope à immersion, du Pluronic® F127, de l'oxyde de Polyethylene, de l'alcool polyvinylique, de l'Hydroxypropylmethyl cellulose, de la gomme de xanthane.

De façon avantageuse et dans la mesure ou le dispositif employé avec la méthode selon l'invention comprend au moins trois orifices d'entrée et trois canaux d'acheminements de ces orifices au canal de séparation, deux solutions tampons différentes sont introduites dans deux orifices d'entrée différent. L'utilisation de deux solutions tampons différentes permet de faciliter le passage de certaines particules ou microorganisme d'un liquide introduit vers un autre durant l'étape de séparation, en jouant notamment sur les différences de densité entre les solutions tampons introduites. Eventuellement, la méthode de traitement selon l'invention peut comprendre une étape préalable de filtration de l'échantillon biologique au travers d'une membrane avant l'introduction dudit échantillon biologique dans le dispositif d'acoustophorèse. Cette étape peut notamment être réalisée par l'introduction de l'échantillon dans un sac comprenant un compartiment délimité par une membrane filtrante, l'échantillon étant prélevé derrière la membrane avant d'être introduit de manière à éliminer les débris les plus gros, préférentiellement les débris d'une taille supérieure à environ 90 $\mu$m.

De façon plus particulière, l'échantillon biologique est introduit dans un dispositif d'acoustophorèse de façon à être acheminé par au moins deux canaux d'acheminement vers le canal de séparation, le dispositif étant agencé pour obtenir un flux laminaire dans le canal de séparation entre les flux d'échantillon et de solution tampon provenant des canaux d'acheminement.

La méthode de traitement selon l'invention permet, à partir d'un échantillon alimentaire non enrichi, de réaliser par exemple une étape de dénombrement de la flore totale de l'échantillon.

A ce titre, l'invention concerne également une méthode de traitement telle que décrite précédemment et comprenant une étape de dénombrement de la flore totale présente dans l'échantillon biologique à la suite de l'étape de séparation. Cette étape peut notamment être réalisée par ensemencement d'un volume de l'échantillon décomplexifié sur un milieu de culture non spécifique, par exemple un milieu Trypticase Soja Agar (TSA ; bioMérieux, France).

De façon avantageuse, la méthode de traitement telle que décrite précédemment peut comprendre la réalisation d'une étape d'enrichissement de l'échantillon biologique, préférentiellement par incubation en présence d'un milieu de culture, avant l'introduction dudit échantillon biologique dans le dispositif d'acoustophorèse. Cette étape d'incubation permet notamment d'augmenter la concentration des espèces d'intérêt susceptibles d'être contenues et éventuellement détectées dans l'échantillon biologique. Le milieu de culture pouvant être employé peut être spécifique ou non spécifique. A titre d'exemple de milieu de culture on peut citer le Bouillon Buffer Peptone Water (BPW ; bioMérieux, France), comme milieu de croissance non spécifique, ou bien les bouillons LX (*Listeria* Xpress broth ; Ref. 42626 bioMérieux, France), LMX (*Listeria Monocytogenes* Xpress broth ; bioMérieux, France ou SX (*Salmonella* Xpress broth ; Ref. 42118 bioMérieux, France) respectivement étudiés pour la croissance spécifique des *Listeria spp./Listeria Monocytogenes et Salmonella.* A titre d'exemple de milieu de culture non spécifique on peut citer le bouillon Trypticase Soja (TSB ; bioMérieux, France).

De façon avantageuse, la méthode de traitement telle que décrite précédemment peut comprendre à la suite de l'étape de séparation par acoustophorèse, la réalisation d'une étape de capture spécifique ou non spécifique de(s) espèce(s) d'intérêt sur un support de capture, suivi d'une étape de concentration par séparation immunologique ou par affinité (« affinity séparation » en langue anglaise). Cette étape de capture peut être réalisée en ligne, également appelé « flux continu », directement à la suite de l'étape de séparation. Dans ce but, l'échantillon biologique décomplexifié est mis en contact avec des supports de capture, éventuellement magnétiques, spécifiques ou non spécifiques de ou des espèce(s) d'intérêt. Cette mise en contact peut être réalisée directement dans l'orifice de sortie contenant l'échantillon décomplexifié. Des supports de capture fonctionnalisés pour la capture spécifique ou non spécifique des analytes recherchés et pouvant être utilisés sont par exemple et à titre non-limitatif: des supports de capture magnétiques (microparticules ou nanoparticules magnétiques), des matériaux poreux ou fibreux, des billes de latex, des résines (échangeuses d'ions, hydrophiliques, hyrdrophobiques,...). Avantageusement, ces supports peuvent être constitués par une des surfaces, notamment par des surfaces horizontales ou verticales de l'orifice de sortie de l'échantillon biologique décomplexifié, celle-ci étant fonctionnalisée.

Cette étape de capture permet notamment de séparer les agents pathogènes de la flore intrinsèque présente dans l'échantillon décomplexifié. L'échantillon décomplexifié étant débarrassé d'une majeure partie des particules non-spécifiques initialement présentes, tel que des débris alimentaires et des inhibiteurs non solubles tel que des particules de gras, le rendement de cette étape de capture est particulièrement amélioré.

Alternativement, cette étape de capture spécifique peut s'effectuer sur des électrodes de Di ElectroPhorese (DEP) déposées sur la surface du dispositif au niveau de l'orifice de sortie. Ces électrodes peuvent être soient fonctionnalisées avec des antigènes de captures dans le cas d'une capture sur un panel d'espèces recherchées, ou non fonctionnalisées utilisant alors uniquement les propriétés bien connues de capture par la charge de la technique DEP. L'identification peut être dans les deux cas de figure confirmée ou effectuée par exemple par identification Raman ou FTIR. Alternativement cette étape de capture générique, sans antigènes, par DEP peut être réversible afin de relarguer les microorganismes dans un volume réduit (environ $20\mu$L) permettant une étape d'identification par spectrométrie de masse telle que par spectrométrie de masse MALDI-TOF.

A titre préférentiel, la méthode de traitement telle que décrite précédemment peut comprendre la réalisation d'une étape d'enrichissement de l'échantillon biologique, préférentiellement par incubation en présence d'un milieu de culture, avant l'introduction dudit échantillon biologique dans le dispositif d'acoustophorèse, et la réalisation d'une étape de lyse suite à l'étape de séparation ou à l'étape de capture sur un support de capture magnétique. Cette étape de lyse permet notamment d'accéder à l'information biologique contenue dans l'espèce(s) d'intérêt. Par information biologique, l'on entend, tout élément constituant ladite espèce d'intérêt ou produit par cette dernière, tel que des acides nucléiques (ADN, ARN), des protéines, des peptides ou des métabolites.

De façon avantageuse, cette étape de lyse peut être suivie d'une étape d'extraction, d'amplification et d'analyse de l'échantillon lysé, préférentiellement par PCR quantitative. Les acides nucléiques obtenus à l'issue de l'étape de lyse sont ainsi détectés et/ou identifiés et/ou quantifiés par toute méthode d'analyse génétique appropriée, par exemple par PCR quantitative (qPCR). Le rendement de ces étapes d'extraction, d'amplification et d'analyse étant particulièrement amélioré par l'étape de séparation par acoustophorèse, celle-ci permettant notamment d'éliminer une partie des inhibiteurs d'amplification.

De façon alternative et à la suite de l'étape de séparation ou de l'étape de capture spécifique, la méthode de traitement selon l'invention peut comprendre la réalisation d'une étape d'analyse de ou des espèce(s) d'intérêt séparées et / ou capturé(es). Cette étape d'analyse est préférentiellement une étape d'identification, préférentiellement par étalement sur un milieu de culture. Des milieux de cultures spécifiques ou non-spécifiques peuvent être employés pour cette étape d'analyse. De façon préférentielle, des milieux sélectifs tel que des milieux chromogéniques peuvent être employés. L'intérêt de combiner ce type d'analyse avec une étape de séparation par acoustophorèse est de pouvoir disposer d'un échantillon décomplexifié pouvant être directement utilisé pour ensemencer le milieu de culture. L'ensemencement du milieu de culture peut être réalisé sans étape intermédiaire, en prélevant l'échantillon décomplexifié dans un orifice de sortie du dispositif. A la suite de l'étape d'analyse, le(s) microorganisme(s) contenu(s) dans l'échantillon biologique peut(vent) être détecté(s) et/ou identifié(s).

Alternativement, cette étape d'analyse est réalisée par un ou des immunoessais spécifique(s) de ou des espèce(s) d'intérêt capturée(s). Cet étape d'analyse permet par exemple de détecter et/ou identifier et/ou quantifier les protéines et/ou les métabolites d'intérêt, initialement présents dans l'échantillon biologique. Ces immunoessais peuvent être notamment spécifiques des supports de capture utilisés dans l'étape de capture et permettent leur détection par un marqueur fluorescent ou enzymatique. Des immunoessais et marqueurs pouvant être utilisés sont par exemple et à titre non-limitatif : des protéines spécifiques du ou des microorganisme(s) recherché(s).

De façon alternative et à la suite de l'étape de séparation par acoustophorèse, la méthode de traitement selon l'invention peut comprendre la réalisation d'une étape de marquage par un marqueur fluorescent non-spécifique ou spécifique de la ou des espèce(s) d'intérêt.

A titre d'exemple de marqueurs fluorescents, on peut citer : des marqueurs des acides nucléiques comme l'iodure de propidium, le SYTO9 ou encore le SYBR® Green, des marqueurs de potentiel membranaire tel que le DiBAC ou encore des dérivés de la fluorescéine couplés à des enzymes spécifiques des microorganismes d'intérêt, qui, une fois clivés à l'intérieur du microorganisme deviennent fluorescents.

A titre d'exemple, d'autres marqueurs peuvent être utilisés parmi la liste ci-dessous :

- des marqueurs des acides nucléiques : TOTO-3, SYTOX Green, Ethidium Bromide, Hoechst 33258/33342, SYTO 13, Mithramycin, Pyronine Y
- des marqueurs protéiques : FITC, Texas Red (sulforhodamine isothiocyanate), Oregon Green isothiocyanate,
- des marqueurs de fonctions cellulaires : Indo-1, Fura-2, Fluor-3,
- des marqueurs dépendants du PH : BCECF, SNARF-1, DIOC6(3),
- des marqueurs de potentiel membranaire : Oxonol, [DiBAC4(3)], Rhodamine 123, Fun-1,
- des marqueurs lipophiles : Nile Red,
- des lectines couplées à des marqueurs fluorescents
- des oligonucléotides couplés à des marqueurs fluorescents
- des substrats couplés à des fluorochromes
- des anticorps couplés à des fluorochromes.

Cette étape de marquage peut être réalisée en ligne, directement à la suite de l'étape de séparation, par la mise en contact de l'échantillon décomplexifié avec une solution contenant des marqueurs fluorescents. Cette mise en contact peut être réalisée directement dans l'orifice de sortie contenant l'échantillon décomplexifié.

A la suite de cette étape de marquage, la méthode de traitement selon l'invention peut comprendre la réalisation d'une étape d'analyse en cytométrie de flux visant à détecter la présence dudit marqueur fluorescent. Cette étape est préférentiellement réalisée en ligne, directement à la suite de l'étape de marquage. Cette étape peut être réalisée en prélevant l'échantillon décomplexifié dans l'orifice de sortie et en l'introduisant dans un cytomètre de flux ou en l'acheminant directement par un dispositif microfluidique. Avantageusement, la solution tampon introduite dans le dispositif pour la réalisation de l'étape de séparation par acoustophorèse peut être compatible avec une étape d'analyse par cytométrie de flux. De façon préférentielle, cette solution tampon peut comprendre des marqueurs fluorescents afin de réaliser directement le marquage des espèces d'intérêt ou de la flore intrinsèque contenue dans l'échantillon biologique.

On entend par échantillon biologique un échantillon d'origine alimentaire, liquide ou visqueux contenant un flore intrinsèque et susceptible de contenir une ou plusieurs espèce(s) d'intérêt, notamment des microorganismes d'intérêt, plus particulièrement un ou des agent(s) pathogène(s). Un échantillon biologique peut présenter une matrice solide ou semi-solide, en suspension dans un liquide. A titre d'exemple d'échantillon d'origine alimentaire, on peut citer des échantillons de viande (poulet, bœuf, steak haché,...), de plats cuisinés, de sauces, de lait, de jus de fruits, de liquide de rinçage de carcasses,..... Les échantillons de poulet sont connus pour être particulièrement complexes à traiter notamment les parties contenant de la peau de poulet telles que les ailes, cuisses ,blancs ainsi que de la peau du cou.

Au sens de la présente invention, le terme microorganisme recouvre les bactéries à Gram positive ou à Gram négative, les levures, les moisissures, les amibes et plus généralement, les organismes unicellulaires, invisibles à l'œil nu, qui peuvent être manipulés et multipliés en laboratoire.

Selon un mode préféré de réalisation de l'invention, le microorganisme est une bactérie, à Gram négatif ou positif, une levure, ou une moisissure.

A titre d'exemple de bactéries à Gram positive, on peut citer les bactéries des genres suivants : *Enterococcus, Streptococcus, Lactobacillus, Bifidobacterium, Staphylococcus, Bacillus, Listeria, Clostridium, Mycobacteria, Nocardia, Corynebacteria, Micrococcus* et *Deinococcus.*

A titre d'exemple de bactéries à Gram négative, on peut citer les bactéries des genres suivants : *Escherichia,* notamment *Escherichia Coli 0157:H7, Enterobacter, Klebsiella, Salmonella, Proteus, Serratia, Campylobacter*

A titre d'exemple de levures, on peut citer les genres suivants : *Candida, Cryptococcus, Saccharomyces* et *Trichosporon.*

A titre d'exemple de moisissures, on peut citer les genres suivants : *Aspergillus, Penicillium, Cladosporium.*

La description présente également- différents dispositifs microfluidiques associés permettant la décomplexification d'échantillons alimentaires, permettant ainsi la capture et/ou l'analyse de microorganismes contenus ou susceptibles d'être contenus dans l'échantillon, lesdits dispositifs microfluidiques étant susceptibles de mettre en œuvre la méthode selon l'invention.

La description présente différents supports pour dispositifs microfluidiques associés permettant la décomplexification d'échantillons alimentaires, permettant ainsi la capture et/ou l'analyse de microorganismes contenus ou susceptibles d'être contenus dans l'échantillon, , lesdits dispositifs microfluidiques étant susceptibles de mettre en œuvre la méthode selon l'invention.

La description présente différents dispositifs de connexion ou connecteurs pour dispositifs microfluidiques associés permettant la décomplexification d'échantillons alimentaires, permettant ainsi la capture et/ou l'analyse de microorganismes contenus ou susceptibles d'être contenus dans l'échantillon, lesdits dispositifs microfluidiques étant susceptibles de mettre en œuvre la méthode selon l'invention.

La description présente enfin un système de régulation d'air pouvant être associé à des dispositifs de connexion ou connecteurs pour dispositifs microfluidiques associés et permettant la décomplexification d'échantillons alimentaires, permettant ainsi la capture et/ou l'analyse de microorganismes contenus ou susceptibles d'être contenus dans l'échantillon, lesdits dispositifs microfluidiques étant susceptibles de mettre en œuvre la méthode selon l'invention..

**Brève description des dessins**

L'invention, sa fonctionnalité, ses applications ainsi que ses avantages seront mieux appréhendés à la lecture de la présente description détaillée qui suit, faite en référence aux figures, dans lesquelles :

- la figure 1 représente une vue de dessus d'un dispositif microfluidique de l'état de l'art, susceptible de mettre en œuvre le procédé selon l'invention,
- la figure 2a représente une vue en coupe longitudinale du dispositif microfluidique de l'état de l'art selon le plan de coupe A-A de la figure 1
- la figure 2b représente une section transversale du canal de séparation 50 selon le plan de coupe B-B de la figure 1
- la figure 3 représente une vue schématique d'un système permettant la mise en œuvre d'une méthode selon la présente invention utilisant un dispositif microfluidique selon l'état de l'art
- la figure 4 représente une vue de dessus d'un premier dispositif microfluidique à deux orifices d'entrée selon l'invention, susceptible de mettre en œuvre le procédé selon l'invention,
- la figure 5a représente une vue en coupe B-B du dispositif microfluidique selon la figure 4
- la figure 5b représente un mode de réalisation d'un orifice d'entrée vue en coupe A-A d'un orifice d'entrée du dispositif microfluidique selon la figure 4.
- la figure 6a représente une vue de dessus d'un second dispositif microfluidique à deux orifices d'entrée selon l'invention, susceptible de mettre en œuvre le procédé selon l'invention,
- la figure 6b représente une vue de dessus d'un troisième dispositif microfluidique à deux orifices d'entrée selon l'invention, susceptible de mettre en œuvre le procédé selon l'invention,

- la figure 6c représente une vue de dessus d'un quatrième dispositif microfluidique à trois orifices d'entrée selon l'invention, susceptible de mettre en œuvre le procédé selon l'invention,
- la figure 6d représente une vue de dessus d'un cinquième dispositif microfluidique à trois orifices d'entrée selon l'invention, susceptible de mettre en œuvre le procédé selon l'invention,
- la figure 7a représente une première alternative de réalisation des orifices de sortie des dispositifs microfluidiques susceptibles de mettre en œuvre la méthode -selon l'invention
- la figure 7b représente une seconde alternative de réalisation des orifices de sortie des dispositifs microfluidiques susceptibles de mettre en œuvre la méthode selon l'invention
- la figure 8 représente une vue en perspective d'un sixième dispositif microfluidique à deux orifices d'entrée, susceptible de mettre en œuvre la méthode selon l'invention,
- la figure 9 représente une vue en perspective d'un premier support pour dispositifs microfluidiques susceptibles de mettre en œuvre la méthode selon l'invention,
- la figure 10 représente une vue en perspective d'un second support pour dispositifs microfluidiques susceptibles de mettre en œuvre la méthode selon l'invention,
- la figure 11 représente une vue de dessus d'un cinquième dispositif microfluidique à deux orifices d'entrée, susceptible de mettre en œuvre la méthode selon l'invention,
- la figure 12a représente une coupe partielle selon le plan A-A de la figure 11
- la figure 12b représente une coupe partielle selon le plan B-B de la figure 11
- la figure 13a représente en vue de dessus un dispositif de connexion pour dispositifs microfluidiques susceptibles de mettre en œuvre la méthode selon l'invention,
- la figure 13b représente le dispositif de connexion selon la coupe A-A de la figure 13a
- la figure 14 représente une vue schématique d'un système de régulation d'air permettant la mise en œuvre d'une méthode selon la présente invention ainsi qu'un dispositif de connexion pour dispositifs microfluidiques susceptibles de mettre en œuvre la méthode selon l'invention,
- la figure 15 est une photographie en vue de dessus d'une partie du dispositif microfluidique du système selon le détail A de la figure 3, au cours de la méthode selon l'invention.
- la figure 16a présente les valeurs de densité optique dans les différents orifices de sortie pour un échantillon de poulet traité selon la méthode de l'invention
- la figure 16b présente les valeurs de densité optique dans les différents orifices de sortie pour un échantillon de bœuf traité selon la méthode de l'invention
- la figure 17 présente les valeurs de décomplexification selon les parties de poulet ou de bœuf traitées selon la méthode de l'invention
- la figure 18 présente les valeurs en Unité Formant Colonie par grammes (CFU.g$^{-1}$) de dénombrement de la flore totale (« total viable count » en langue anglaise) selon la méthode de l'invention
- la figure 19a présente les valeurs en Unité Formant Colonie par millilitre (CFU.ml$^{-1}$) de dénombrement de *Salmonella Typhimurium* selon la méthode de l'invention pour des échantillons de poulet
- la figure 19b présente les valeurs en Unité Formant Colonie par millilitre (CFU.ml$^{-1}$) de dénombrement de *Salmonella Typhimurium* selon la méthode de l'invention pour des échantillons de bœuf
- la figure 20 présente les valeurs de décomplexification pour des échantillons de poulet ou de bœuf traités selon la méthode de l'invention
- la figure 21 présente les valeurs en Unité Formant Colonies par millilitre (CFU.ml$^{-1}$) de dénombrement de *Salmonella Typhimurium* selon la méthode de l'invention pour des échantillons de poulet et de bœuf

La description détaillée ci-après a pour but d'exposer l'invention de manière suffisamment claire et complète, notamment en référence aux figures susvisées, mais ne doit en aucun cas être regardée comme limitant l'étendue de la protection aux modes de réalisation particuliers objets desdites figures.

Les procédés de préparations d'échantillons biologiques ou chimiques complexes font appels à des opérations de séparations des particules, cellules ou molécules afin de rendre possible ou de faciliter l'analyse de particules, cellules ou molécules d'intérêts, susceptibles d'être contenues dans l'échantillon. Un objectif de ces procédés de préparations d'échantillons et donc de séparer et/ou de concentrer les particules, cellules ou molécules d'intérêt vis à vis d'éléments non-spécifiques pour permettre par exemple leur capture et/ou leur détection.

Parmi les procédés classiques de séparation de particules, la centrifugation, la filtration, la chromatographie ou l'électrophorèse ont étés largement utilisés pour la préparation d'échantillon biologiques ou chimiques complexes. Cependant, ces méthodes sont souvent fastidieuses à mettre en œuvre et ne permettent pas de traiter de grands volumes d'échantillons. Par exemple, les procédés de filtration obligent de traiter un volume défini d'échantillon puis de procéder à une opération de nettoyage du filtre pour éviter tout bouchage. Cette technique ne permet donc pas de traiter un échantillon biologique ou chimique complexe en flux continu. D'autre part, des techniques classiques de préparation d'échantillon biologique, notamment la lyse chimique ou la lyse sélective, ne permettent pas d'assurer ou peuvent limiter la viabilité des cellules vivantes telle que des bactéries suite à la préparation de l'échantillon, et peuvent donc impacter la qualité des étapes de capture, de remise en croissance et/ou de détection ultérieures.

Des procédés de séparation de particules, cellules ou molécules en flux continu utilisant des dispositifs microfluidiques peuvent permettre de traiter des grands volumes d'échantillon et ce en introduisant continuellement l'échantillon dans le dispositif microfluidique. Un autre intérêt de ces techniques et leur potentiel d'intégration en amont ou en aval d'une étape de capture ou d'analyse de l'échantillon, assurant ainsi un rôle d'aiguillage et/ou de filtre dans une chaine de traitement et d'analyse d'un échantillon biologique ou chimique complexe.

Un certain nombre de forces ont été utilisées avec succès dans des dispositifs microfluidiques, y compris les forces d'inertie, électriques, magnétiques ainsi que les forces mécaniques de contact. Parmi ces diverses forces appliquées pour la séparation de particules, cellules ou molécules dans des dispositifs microfluidiques, les forces acoustiques générées à partir d'ondes ultrasonores ont également été largement utilisées pour la séparation des particules de taille micrométrique en suspension, pour les séparer de leur milieu et/ou d'autres particules. Cette technique, dite d'acoustophorèse, permet la séparation non destructive et sans marqueur, uniquement sur la base de la taille, la densité et la compressibilité de particules, cellules ou molécules.

L'acoustophorèse consiste en l'application d'une onde acoustique stationnaire sur un ou plusieurs canaux d'un dispositif microfluidique, présentant ainsi un profil de pression immobile et disposé transversalement vis à vis du canal visé. Le profil de pression des ondes acoustiques stationnaires appliquées varie ainsi entre des zones de haute pression, appelées des nœuds (« nodes » en langue anglaise), et des zones de basse pression, appelées anti nœuds (« anti-nodes » en langue anglaise). De façon classique, plusieurs fluides de densités identiques sont introduits par des canaux d'acheminement dans un dispositif microfluidique de façon à s'écouler de façon laminaire (donc sans mélange) dans le canal de séparation, en regard d'un transducteur ultrasonore piézoélectrique. Quand le transducteur n'est pas excité par un signal de commande, les fluides introduits s'échappent du canal de séparation sans observer de mélange ni de migration d'un fluide vers l'autre. En appliquant un signal de commande au transducteur, les particules présentes dans les différents fluides vont subir la force acoustique ainsi générée et se déplacer vers les nœuds de pression ou les anti-nœuds en fonction de leur taille, leur densité et leur compressibilité. La force acoustique s'appelle également pression de radiation acoustique. La densité des fluides introduits ainsi que leur compressibilité influencent également. Cette migration des particules dans le canal de séparation permet donc de favoriser leur concentration dans certain canaux d'acheminement et vers les orifices de sorties du dispositif microfluidique, en aval de canal de séparation.

L'amplitude de la radiation acoustique générée par le transducteur est proportionnelle à celle du signal de commande appliqué, cependant, les effets maximums de la force acoustique sont obtenus à partir d'un même signal de commande quand la fréquence et l'amplitude de celui-ci font entrer le dispositif microfluidique en résonance. Pour une configuration à un seul nœud de pression, cette fréquence de résonance est dépendante de la largeur du canal de séparation ainsi que du matériau dans lequel est fabriqué le dispositif. Des matériaux classiques pouvant être employés sont le verre ou le silicium, ceux-ci présentant des surfaces idéales de réflexion des ondes acoustique.

La pression de radiation dû à l'onde acoustique influence majoritairement les particules de taille supérieures à $2\mu m$. Cette pression étant directement proportionnelle au volume des particules, un changement mineur du rayon de la particule diminue ou augmente rapidement son impact sur celle-ci.

Une autre force est également créée par une onde ultrasonore stationnaire dans un canal contenant une suspension de microparticules. Cette force est due à la diffusion et la réflexion de l'onde acoustique dans le fluide et sur les particules. La force de diffusion acoustique est relativement faible et affecte surtout les particules inférieures à $2\mu m$.

Un dispositif de séparation par acoustophorèse peut donc être créé par l'utilisation d'un transducteur acoustique ultrasonore en regard d'un surface de réflexion ou d'un second transducteur de manière à établir une onde stationnaire résonante dans le canal de séparation.

Un dispositif microfluidique comprend ainsi au moins deux orifices d'entrée, un canal de séparation et au moins deux orifices de sortie. Les orifices d'entrée débouchent vers le canal de séparation, tandis que le canal de séparation débouche vers les orifices de sortie. Le dispositif est agencé pour qu'un transducteur à ultrasons puisse être intégré dans ou accolé à une paroi dudit canal de séparation. Le transducteur à ultrasons ainsi intégré ou accolé est apte à transmettre des oscillations mécaniques en ondes acoustiques multiples agissant sur le contenu du canal de séparation.

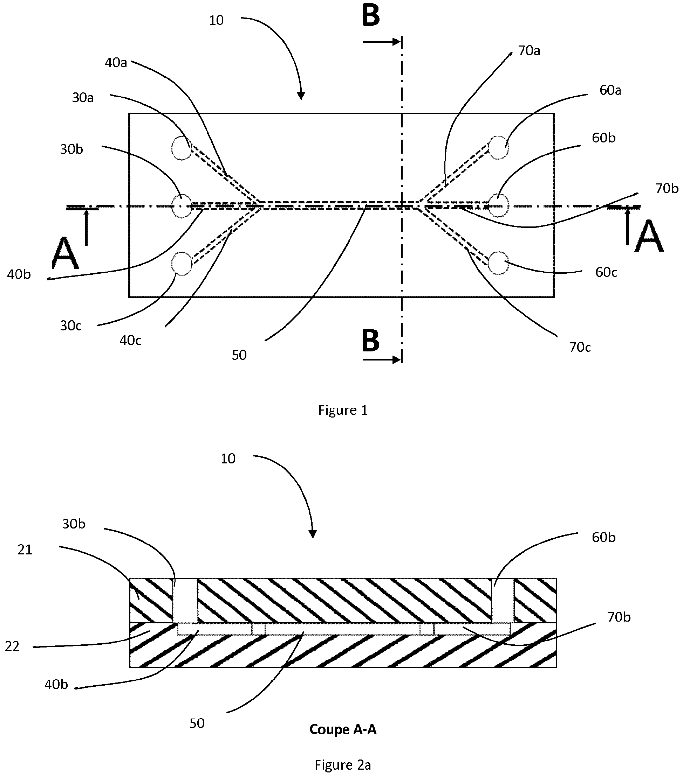

Comme représenté sur les figures 1 et 2a, un dispositif microfluidique 10 de l'art antérieur comprend deux parties fluidiques 21, également appelée plaque de recouvrement, et 22, également appelé séparateur. Le séparateur et la plaque de recouvrement sont sensiblement plans, et assemblés entre eux de manière à réaliser des canaux fluidiques. Le séparateur est lié à la plaque de recouvrement pour réaliser un dispositif 10 apte à être observé par un dispositif d'analyse optique tel qu'un microscope.

Le dispositif se compose de trois orifices d'entrées 30a, 30b, 30c et de trois orifices de sorties 60a, 60b, 60c reliés par un canal de séparation 50 rectiligne, d'une longueur de 35 mm, permettant, lors du fonctionnement du système, aux cellules, particules ou molécules de devenir acoustiquement concentrés dans le canal d'acheminement 70b, dont l'axe est identique et dans le prolongement de l'axe du canal de séparation 50. Les cellules, particules ou molécules sont acoustiquement concentrées dans le canal central en fonction de leur densité, leur taille et leur compressibilité.

Les trois orifices d'entrée 30a, 30b, 30c, communiquent avec le canal de séparation 50 par le biais de canaux d'acheminement respectivement 40a, 40b, 40c. Le canal de séparation 50 communique également avec les trois orifices de sortie 60a, 60b, 60c, par le biais de canaux d'acheminement, respectivement 70a, 70b, 70c.

Le séparateur 22 comprend une plaquette de verre de 1mm d'épaisseur revêtue d'une couche de chrome et d'une couche de résine photosensible. Après développement de la couche photosensible, le verre est gravé par de l'acide fluorhydrique (HF) produisant des canaux d'acheminement et de séparation d'une profondeur de 125 μm, d'une largeur inférieure, au fond des canaux, de 375 μm, et d'une largeur supérieure de 625 μm dans le plan du séparateur en contact avec la plaque de recouvrement. La plaque de recouvrement 21 est percée pour réaliser les orifices d'entrée et de sorties puis collée thermiquement avec le séparateur afin de réaliser un assemblage étanche.

Les canaux d'acheminement des orifices d'entrée 40a, 40b, 40c présentent un angle de respectivement de 45°, 0° et moins 45° par rapport au canal de séparation dans le plan du séparateur, de manière à ralentir le débit des fluides provenant des canaux d'acheminement présentant un angle de 45 ° ou -45° , 40a, 40c, par rapport au canal de séparation et de favoriser ainsi l'apparition d'un flux laminaire dans le canal de séparation.

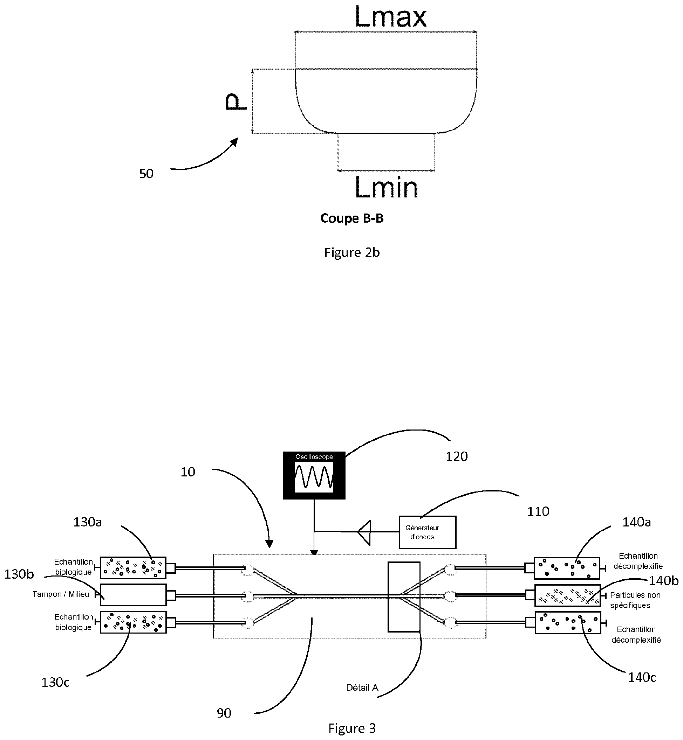

Le profil type d'un canal d'acheminement ou de séparation est représenté sur la figure 2b. Les canaux présentent une forme de D dans le plan perpendiculaire à l'axe du canal considéré, la largeur la plus importante étant située dans le plan en contact avec la plaque de recouvrement quand le dispositif est assemblé. Ils présentent ainsi une largeur inférieure « Lmin », au fond des canaux, une largeur supérieure « Lmax » dans le plan du séparateur en contact avec la plaque de recouvrement quand le dispositif est assemblé ainsi qu'une profondeur « P ».

Ce dispositif de l'art antérieur est susceptible de mettre en œuvre des procédés de préparation d'échantillons biologiques par acoustophorèse tel que le procédé selon l'invention. Pour cela, tout ou partie de l'échantillon biologique à traiter, est introduit dans les orifices d'entrée 30a et 30c du dispositif 10. Une solution tampon est introduite dans l'orifice d'entrée 30b, le tampon et l'échantillon étant introduits à des débits respectifs aptes à générer un flux laminaire dans le canal de séparation. Un transducteur ultrasonore, tel qu'un transducteur piézoélectrique, accolé au canal de séparation est alors activé par un signal de commande, de manière à réaliser une étape de séparation dudit échantillon biologique par acoustophorèse. Cette séparation permet de favoriser la concentration des particules non-spécifiques, tel que des débris alimentaires, présents dans l'échantillon, dans l'orifice de sortie 60b dudit dispositif d'acoustophorèse. L'échantillon décomplexifié étant obtenu dans les orifices de sortie 60a et 60c.

L'invention concerne une méthode applicable à différents dispositifs microfluidiques permettant le traitement d'échantillons biologiques par acoustophorèse. Ces différents dispositifs sont également susceptibles de mettre en œuvre le procédé selon l'invention de façon avantageuse. Ces dispositif sont particulièrement intéressants à mettre en œuvre avec le procédé selon l'invention car ils permettent d'atteindre des débits de traitement d'échantillons plus élevés que des dispositifs de l'art antérieur tout en assurant un taux de décomplexification égal ou supérieur à ceux observé dans l'art antérieur pour des échantillons biologique tel que des échantillons alimentaires. D'autre part ces dispositifs selon l'invention permettent de traiter une grande variété d'échantillons alimentaires, la taille de leurs canaux, notamment de leurs canaux de séparation étant optimisée pour les différentes tailles de particules non spécifiques ou débris pouvant être observés.

Les orifices d'entrée et la forme des canaux d'acheminements communiquant avec les orifices d'entrée peuvent avantageusement être optimisés afin d'accepter une sédimentation des particules non spécifiques ou débris aux entrées du dispositif microfluidique sans risque de bouchage des canaux du dispositif. A ce titre, la partie de chaque canal d'acheminement en regard de chaque orifice d'entrée susceptible de recevoir l'échantillon biologique peut présenter une cavité d'une profondeur plus importante que la profondeur générale du canal d'acheminement. Avantageusement, la cavité en regard de l'orifice d'entrée est deux à trois fois plus profonde que le canal d'acheminement connecté audit orifice. Cette cavité permet de créer une zone de vitesse ralentie ou nulle de l'échantillon introduit, favorisant la sédimentation des particules non-spécifiques. Le dispositif ainsi formé présente une meilleure tolérance aux bouchages liés à l'introduction de débris de taille et de densité importantes.

Pour chacun de ces modes de réalisation, le séparateur peut être réalisé en silicium, ceramique ou en verre, les canaux étant obtenus par gravure chimique ou physique (e.g. sablage). Le séparateur peut également être réalisé en matériau polymère, souple et fin tel que du polydiméthylsiloxane (PDMS), du Polypropylène ou un bicomposé Polymère / Silicone (par exemple un Polystyrène avec un film souple de silicone ou de PDMS afin d'assurer un couplage sans air des transducteurs piézoélectriques sur la paroi du dispositif), les canaux étant alors obtenu par moulage. L'avantage de l'utilisation d'un matériau polymère, souple et fin et de pouvoir réaliser un dispositif à très bas coût, permettant son utilisation comme consommable en routine dans des procédé de préparation d'échantillons biologiques par acoustophorèse tel que le procédé selon l'invention. Un autre avantage étant de ne pas nécessiter de gel de couplage ou de colle entre le/les transducteurs piézoélectrique(s) et les parois du canal de séparation.

Pour chacun de ces modes de réalisation, chaque orifice, d'entrée ou de sortie, peut former un réservoir de manière à pouvoir réaliser des opérations de stockage d'un échantillon ainsi que des opérations de dépose ou de pipetage. En accolant un moyen de chauffage à un orifice, une étape d'incubation de l'échantillon peut également être réalisée. De façon avantageuse, un orifice peut être réalisé dans un matériau transparent ou translucide, de manière à pouvoir réaliser directement une lecture de la densité optique de l'échantillon qu'il contient.

Pour chacun de ces modes de réalisation, chaque dispositif peut être utilisé seul ou en parallèle de manière à pouvoir traiter un même volume d'échantillon biologique à un débit plus important. En effet, si on augmente le débit d'introduction des fluides dans un même canal de séparation, le taux de décomplexification de l'échantillon peut décroitre rapidement, les particules n'ayant pas le temps d'être séparées par l'onde acoustique dû à un temps de résidence trop court dans le dispositif. D'autre part, si la taille des canaux, notamment du canal de séparation est augmentée, dans l'espoir de pouvoir également augmenter le débit de traitement, l'amplitude d'excitation (du signal de commande) du/des transducteur(s) piézoélectrique(s) nécessaire à une bonne séparation des particules devra être bien plus importante puisque la fréquence de résonance sera réduite et par conséquence la pression de radiation sur les particules à focaliser au centre du canal de séparation. Cependant, en excitant le transducteur à ultrasons à une plus forte amplitude, celui-ci peut provoquer un échauffement local dans le canal de séparation. Cet échauffement n'est pas souhaitable car celui-ci peut engendrer une dégradation du dispositif ainsi que de la viabilité des particules, cellules, ou molécules d'intérêt présentes dans le canal de séparation. D'autre part, cet échauffement peut engendrer un changement de densité du tampon utilisé ce qui peut modifier la propagation des ondes acoustiques dans le canal et perturber l'étape de séparation. De ce fait les dispositifs selon l'invention, utilisés seul ou en parallèle, permettent d'obtenir des débits de séparation / décomplexification égaux ou supérieurs à ceux de l'art antérieur tout en garantissant la viabilité des particules, cellules, ou molécules d'intérêt traitées.

Ainsi les canaux de séparation des différents dispositifs selon l'invention présentent une longueur pouvant être comprise entre 35mm et 80mm. Les largeurs inférieures du canal de séparation sont par exemple comprises entre $300\mu m$ et $375\mu m$, les largeurs supérieures sont par exemple comprises entre et $550\mu m$ et $625\mu m$. La profondeur du canal de séparation peut être comprise entre $100\mu m$ et $150\mu m$, préférentiellement de $125\mu m$. Les différents dispositifs permettant la mise en œuvre de la méthode selon l'invention sont adaptés pour être utilisés avec des signaux de commandes de(s) transducteur(s) ultrasonore(s) accolé(s) présentant une fréquence comprise entre 300kHz et 10MHz, préférentiellement de 1,3MHz alternativement de 1,44MHz pour la création d'un seul nœud de pression au centre du canal. Ces différentes valeurs de fréquence permettent d'obtenir une focalisation centrale (un seul nœud de pression dans le canal de séparation) présentant des particules non-spécifiques focalisées dans l'orifice de sortie dans le prolongement de l'axe du canal de séparation ou de privilégier la concentration de ces particules non-spécifiques dans un des orifices de sortie. Différentes fréquences de résonance peuvent être observées pour un même dispositif du fait de l'apparition de plusieurs nœuds de pression dans la largeur du canal de séparation en multiple du quart de la longueur d'onde ($n\lambda/4$). Des variations légères d'environ 30kHz autour de la fréquence de résonance peuvent également permettre d'obtenir une meilleure résonance du dispositif, cette fréquence étant dépendante de la qualité de réalisation des canaux du dispositif.

L'amplitude du signal de commande est comprise entre 0,1V et 100V, préférentiellement de 38V. Pour des amplitudes du signal de commande supérieures à 38V, il peut être souhaitable d'utiliser un dispositif de refroidissement accolé au(x) transducteur(s) à ultrasons pour empêcher une dégradation du dispositif et/ou une atteinte à la viabilité des particules, cellules, ou molécules présentes dans le canal de séparation. Des blocs Peltier ou des ventilateurs peuvent constituer de tels dispositifs de refroidissement. Avantageusement, le dispositif de refroidissement est asservi en température afin de réguler la température à proximité du transducteur ultrasonore.

Pour chacun de ces modes de réalisation, les canaux d'acheminement des orifices d'entrée utilisés pour l'introduction de l'échantillon biologique présentent un angle compris entre 30 et 60°, préférentiellement de 45° par rapport au canal de séparation dans le plan du séparateur. Cet angle peut aisément être adapté par l'homme du métier de manière à ralentir plus ou moins le débit d'introduction du fluide dans le canal de séparation, et assurer ainsi l'apparition d'un écoulement laminaire des fluides dans ce canal. Ces canaux d'acheminement sont appelés canaux latéraux car ils permettent d'introduire le fluide en direction des parois du canal de séparation.

Pour chacun de ces modes de réalisation, les canaux d'acheminement vers les orifices de sortie utilisé pour récupérer ou réintroduire l'échantillon décomplexifié présentent un angle compris entre 30 et 60°, préférentiellement de 45° par rapport au canal de séparation dans le plan du séparateur. Cet angle peut aisément être adapté par l'homme du métier de manière à ralentir plus ou moins le débit d'aspiration du fluide hors du canal de séparation, et assurer ainsi l'apparition d'un écoulement laminaire des fluides dans ce canal. Ces canaux d'acheminement sont également appelés canaux latéraux car ils permettent d'aspirer le fluide circulant le long des parois du canal de séparation.

Les canaux d'acheminements dont l'(les) axes est (sont) identique(s) à l'axe du canal de séparation sont appelés canaux centraux. Ces canaux transportent l'échantillon concentré.

D'autre part les sections des canaux d'acheminement des orifices d'entrée et de sortie peuvent être adaptées. De façon particulière, les sections des canaux d'acheminement des orifices de sortie latérales (dans lesquels on retrouve l'échantillon décomplexifié) par rapport à la section du canal d'acheminement de l'orifice de sortie central (dans lequel on retrouve un maximum de particules non-spécifiques) peuvent être adaptées afin de garantir une meilleure focalisation au niveau de l'embranchement de sortie entre ces canaux. Dans cette zone, à la jonction du canal de séparation et des canaux d'acheminement vers les orifices de sortie, la résonance est peu effective (car il n'y a plus de parois latérales à l'embranchement) durant un temps transitoire court. Ceci conduit à une perte partielle de focalisation des particules non-spécifiques. Dans le cas du dispositif illustré sur la figure 1, l'ajustement des rapports des sections entre les canaux 70a ou 70c et 70b peut ainsi permettre de minimiser les pertes de microorganismes ou d'améliorer le taux de concentration des particules non-spécifiques ou débris dans le canal d'acheminement central 70b.

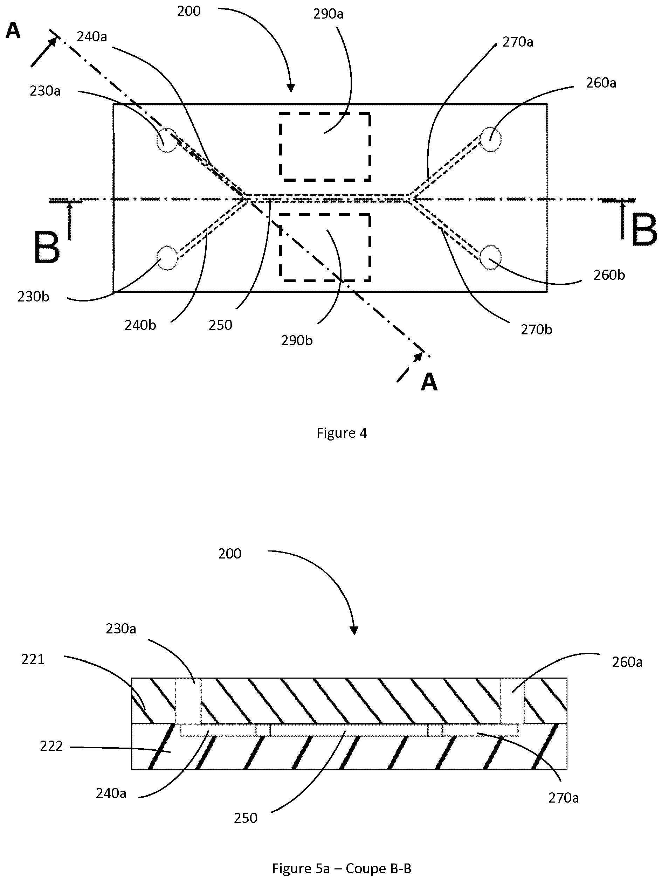

Un premier mode de réalisation d'un dispositif permettant la mise en œuvre de la méthode selon l'invention est représenté sur les figures 4 et 5a. Ce dispositif 200 comprend deux orifices d'entrée 230a, 230b, un canal de séparation 250 et au moins deux orifices de sorties 260a, 260b. Le dispositif 200 comprend deux parties fluidiques, un séparateur 222 et une plaque de recouvrement 221, sensiblement planes. Ces deux parties sont assemblées de façon hermétique. Les orifices d'entrée débouchent vers le canal de séparation, tandis que le canal de séparation débouche vers les orifices de sortie par le biais de canaux d'acheminement. Ainsi, le canal de séparation 250 débouche vers l'orifice 230a via le canal d'acheminement 240a, vers l'orifice 230b via le canal d'acheminement 240b, vers l'orifice 260a via le canal d'acheminement 270a, vers l'orifice 260b via le canal d'acheminement 270b. Dans une première alternative de réalisation de ce premier mode de réalisation, le dispositif est agencé pour qu'au moins un transducteur à ultrasons puisse être intégré dans ou accolé à une paroi dudit canal de séparation. Le transducteur à ultrasons ainsi intégré ou accolé est apte à transmettre des oscillations mécaniques en ondes acoustiques multiples pouvant agir sur le contenu du canal de séparation.

Dans une seconde alternative de réalisation de ce premier mode de réalisation, le dispositif comprend deux évidements 290a, 290b, réalisés le long du canal de séparation 250, dans le séparateur 222, et susceptibles de recevoir chacun un transducteur à ultrasons. Ce mode de réalisation permet l'obtention d'une onde stationnaire dans un séparateur réalisé dans un matériau peu réfléchissant aux ondes acoustiques, le matériau étant souple et fin tel que des matériaux plastiques, polymères, silicones. Un silicone préférentiellement utilisé est le Polydiméthylsiloxane (PDMS). Préférentiellement, chaque transducteur à ultrasons comprend une lame ¼ onde adaptatrice dont l'impédance acoustique est calculée pour minimiser les pertes d'énergie, et donc l'échauffement, entre le transducteur à ultrasons et les parois du dispositif. Cette lame de résine recouvrant le/les transducteur(s) piézoélectrique(s) (réalisée par coulage et polymérisation), est définie pour avoir une impédance acoustique intermédiaire entre l'impédance acoustique du matériau utilisé pour la réalisation du dispositif, et l'impédance acoustique du(es) transducteur(s) piézoélectrique(s).

Préférentiellement la plaque de recouvrement 221 est réalisée en Polydiméthylsiloxane (PDMS) ou en matériau plastique moulé tel que du Polycarbonate (PC), poly(méthacrylate de méthyle) (PMMA), polypropylène (PP), Polystyrène (PS), Acrylonitrile butadiène styrène (ABS), Copolymère d'Oléfine Cyclique (COC), Polymère d'Oléfine Cyclique (COP), Polyoxyméthylène (POM).

Ce dispositif 200 est susceptible de mettre en œuvre des procédés de préparation d'échantillons biologiques par acoustophorèse tel que la méthode selon l'invention. Pour cela, tout ou partie de l'échantillon biologique à traiter, est introduit dans l'orifice d'entrée 230a du dispositif 200. Une solution tampon est introduite dans l'orifice d'entrée 230b, le tampon et l'échantillon étant introduits à des débits respectifs aptes à générer un flux laminaire dans le canal de séparation. Un transducteur ultrasonore, tel qu'un transducteur piézoélectrique, accolé au canal de séparation est alors activé par un signal de commande, de manière à réaliser une étape de séparation dudit échantillon biologique par acoustophorèse. Cette séparation permet de favoriser la concentration des particules non-spécifiques, tel que des débris alimentaires, présents dans l'échantillon, dans l'orifice de sortie 260b dudit dispositif d'acoustophorèse. L'échantillon décomplexifié étant obtenu dans l'orifice de sortie 260a. Les particules non spécifiques sont ainsi transférées dans la solution tampon introduite dans l'entrée 230b.

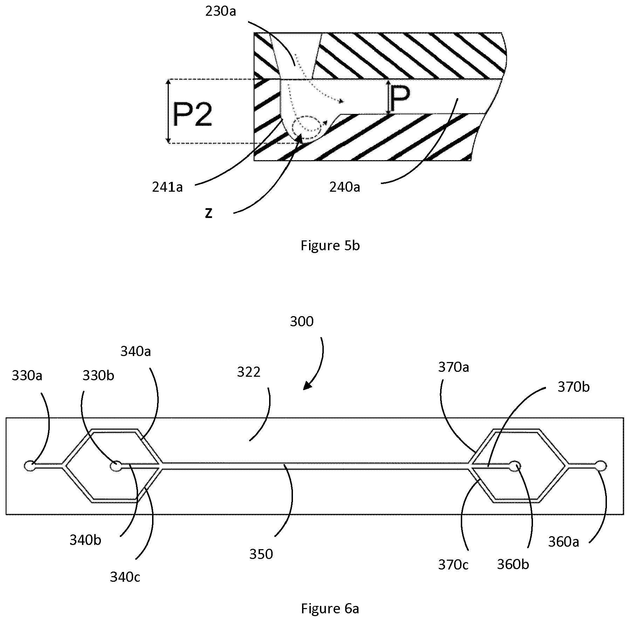

Une alternative de réalisation de l'orifice d'entrée 230a est présentée sur la figure 5b. L'orifice d'entrée 230a présente ici une forme tronconique mais peut également être cylindrique en fonction du moyen d'introduction choisi. La forme du canal d'acheminement 240a, en regard de l'orifice 230a est modifiée de manière à comprendre une cavité 241a de profondeur plus importante que la profondeur générale du canal d'acheminement 240a. Cette cavité présente ainsi une profondeur P2 avec P2 comprise entre deux et trois fois la profondeur P du canal d'acheminement. Cette cavité permet de créer une zone de vitesse ralentie ou nulle (Z) de l'échantillon introduit, favorisant la sédimentation des particules non-spécifiques contenues dans l'échantillon. Le dispositif ainsi formé présente une meilleure tolérance aux bouchages liés à l'introduction de débris de taille et de densité importantes.

Comme représenté sur la figure 6a, l'invention concerne également un second mode de réalisation d'un dispositif 300, susceptible de mettre en œuvre la méthode selon l'invention, -comprenant deux parties fluidiques, un séparateur 322 et une plaque de recouvrement (non représentée), ces deux parties étant sensiblement planes. Ces deux parties sont assemblées de façon hermétique. Des canaux sont réalisés dans le séparateur 322. Le dispositif 300 comprend deux orifices d'entrée 330a, 330b, en communication fluidique avec au moins un canal de séparation 350 par le biais de canaux d'acheminement respectivement 340a, 340c pour l'orifice 330a et 340b pour l'orifice 330b. Le canal de séparation 350 est également en communication fluidique avec deux orifices de sortie 360a, 360b, par le biais de canaux d'acheminement, respectivement 370a et 370c pour l'orifice 360a et 370b pour l'orifice 360b. Cette configuration permet de simplifier la connexion fluidique car elle présente uniquement deux orifices d'entrée et deux orifices de sortie. De même, la collecte de l'échantillon décomplexifié peut être réalisée dans un orifice de sortie ou un tube de collection unique.

D'autre part, les canaux d'acheminement 370a et 370c vers l'orifice de sortie 360a étant directement connectés par gravure des canaux sur le dispositif, l'équilibrage des pertes de charge au niveau de l'embranchement des canaux d'acheminement vers les orifices de sortie est significativement améliorée. Une différence minime de perte de charge (liée à une différence de connexion des tubes de sortie sur le dispositif) pouvant entrainer une déviation de la focalisation et ainsi une dégradation importante des performances de décomplexification.

Le dispositif 300 est agencé pour qu'un transducteur à ultrasons, non représenté, puisse être intégré dans ou accolé à une paroi dudit canal de séparation 350. Le transducteur à ultrasons ainsi intégré ou accolé est apte à transmettre des oscillations mécaniques en ondes acoustiques multiples pouvant agir sur le contenu du canal de séparation et y générer une onde acoustique stationnaire. Un générateur de courant alternatif associé à un amplificateur de signal, non représentés, peut être connecté électriquement au transducteur afin de générer un signal de commande du transducteur dont la fréquence, la forme d'onde et l'amplitude sont connues.

Dans un mode de réalisation particulier du dispositif 300, le séparateur 322 est réalisé dans une plaquette de verre de 1mm d'épaisseur revêtue d'une couche de chrome et d'une couche de résine photosensible. Après développement de la couche photosensible, le verre est gravé par de l'acide fluorhydrique (HF). Les canaux d'acheminement 340a et 340c, 370a et 370c présentent une profondeur P de 125 $\mu$m, une largeur inférieure Lmin, au fond des canaux, de 300 $\mu$m, et d'une largeur supérieure Lmax de 550 $\mu$m dans le plan du séparateur en contact avec la plaque de recouvrement. Les canaux d'acheminement 340b et 370b ainsi que le canal de séparation 350 présentent une profondeur P de 125 $\mu$m, une largeur inférieure Lmin, au fond des canaux, de 375 $\mu$m, et une largeur supérieure Lmax de 625$\mu$m dans le plan du séparateur en contact avec la plaque de recouvrement. La plaque de recouvrement est percée pour réaliser les orifices d'entrée et de sortie puis collée thermiquement avec le séparateur afin de réaliser un assemblage étanche. Les canaux d'acheminement des orifices d'entrée 340a, 340b, présentent un angle de respectivement de 45°, 0° et moins 45° par rapport au canal de séparation 350 dans le plan du séparateur, de manière à ralentir le débit des fluides provenant des canaux d'acheminement présentant un angle de 45 ° ou -45°, 340a, 340c, par rapport au canal de séparation. Le canal de séparation 350 rectiligne, présente une longueur de 80 mm, permettant, aux cellules, particules ou molécules de devenir acoustiquement concentrés dans le canal d'acheminement 370b lors du fonctionnement du système. L'axe du canal d'acheminement 370b est identique à l'axe du canal de séparation. Les cellules, particules ou molécules sont acoustiquement concentrées dans le canal central 370b, en fonction de leur densité, leur taille et leur compressibilité. Alternativement, le séparateur 322 du dispositif 300 comprend deux évidements (non représentés) réalisés le long du canal de séparation 350, et susceptibles de recevoir chacun un transducteur à ultrasons (non représentés). Ce mode de réalisation permet l'obtention d'une onde stationnaire dans le séparateur 322 si celui-ci réalisé dans un matériau peu réfléchissant aux ondes acoustiques, le matériau étant souple et fin tel que des matériaux plastiques, polymères, silicones. Un silicone préférentiellement utilisé est le Polydiméthylsiloxane (PDMS). Préférentiellement, chaque transducteur à ultrasons comprend une lame ¼ onde adaptatrice dont l'impédance acoustique est calculée pour minimiser les pertes d'énergie, et donc l'échauffement, entre le transducteur à ultrasons et les parois du dispositif.

Préférentiellement la plaque de recouvrement (non représentée) est réalisée en PDMS ou en matériau plastique moulé tel que du Polycarbonate (PC), poly(méthacrylate de méthyle) (PMMA), polypropylène (PP), Polystyrène (PS), Acrylonitrile butadiène styrène (ABS), Copolymère d'Oléfine Cyclique (COC), Polymère d'Oléfine Cyclique (COP), Polyoxyméthylène (POM).

Ce dispositif 300 est susceptible de mettre en œuvre des procédés de préparation d'échantillons biologiques par acoustophorèse tel que la méthode selon l'invention. Pour cela, tout ou partie de l'échantillon biologique à traiter, est introduit dans l'orifice d'entrée 330a du dispositif 300. Une solution tampon est introduite dans l'orifice d'entrée 330b, le tampon et l'échantillon étant introduits à des débits respectifs aptes à générer un flux laminaire dans le canal de séparation. Un transducteur ultrasonore, tel qu'un transducteur piézoélectrique, accolé au canal de séparation est alors activé par un signal de commande, de manière à réaliser une étape de séparation dudit échantillon biologique par acoustophorèse. Cette séparation permet de favoriser la concentration des particules non-spécifiques, tel que des débris alimentaires, présents dans l'échantillon, dans l'orifice de sortie 360b dudit dispositif d'acoustophorèse. L'échantillon décomplexifié étant obtenu dans l'orifice de sortie 360a.

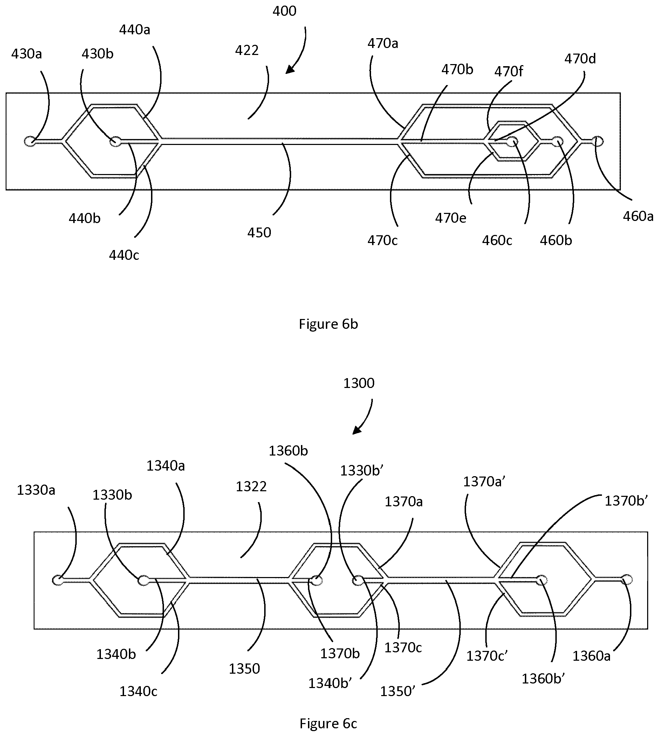

Comme représenté sur la figure 6b, l'invention concerne également un troisième dispositif 400 comprenant deux parties fluidiques, un séparateur 422 et une plaque de recouvrement (non représentée), ces deux parties étant sensiblement planes. Ces deux parties sont assemblées de façon hermétique. Des canaux fluidiques sont réalisés dans le séparateur 422. Le dispositif 400 comprend deux orifices d'entrée 430a, 430b, en communication fluidique avec au moins un canal de séparation 450 par le biais de canaux d'acheminement respectivement 440a, 440c, pour l'orifice 430a et 440b pour l'orifice 430b. Le canal de séparation 450 est également en communication fluidique avec trois orifices de sortie 460a, 460b, 460c, par le biais de canaux d'acheminement, respectivement 470a et 470c pour l'orifice 460a , 470b et 470d pour l'orifice 460c ; 470b, 470f et 470e pour l'orifice de sortie 460b. Le dispositif 400 est agencé pour qu'un transducteur à ultrasons, non représenté, puisse être intégré dans ou accolé à une paroi dudit canal de séparation 450. Le transducteur à ultrasons ainsi intégré ou accolé est apte à transmettre des oscillations mécaniques en ondes acoustiques multiples pouvant agir sur le contenu du canal de séparation et y générer une onde acoustique stationnaire. Un générateur de fonction associé à un amplificateur de signal, non représentés, peut être connecté électriquement au transducteur afin de générer un signal de commande du transducteur dont la fréquence, la forme d'onde et l'amplitude sont connues. Le canal d'acheminement 470b, disposé dans le prolongement du canal de séparation 450, permet la réalisation d'une seconde étape de séparation des particules non-spécifiques pouvant subsister dans l'échantillon biologique.

Dans un mode de réalisation particulier du dispositif 400, le séparateur 422 est réalisé dans une plaquette de verre de 1mm d'épaisseur revêtue d'une couche de chrome et d'une couche de résine photosensible. Après développement de la couche photosensible, le verre est gravé par de l'acide fluorhydrique (HF). Les canaux d'acheminement 440a, 440c, 470a, 470c, 470e et 470f présentent une profondeur P de 125 $\mu$m, une largeur inférieure Lmin, au fond des canaux, de 300 $\mu$m, et une largeur supérieure Lmax de 550 $\mu$m dans le plan du séparateur en contact avec la plaque de recouvrement.

Les canaux d'acheminement 440b, 470b et 470d ainsi que le canal de séparation 450 présentent une profondeur P de 125 $\mu$m, une largeur inférieure Lmin, au fond des canaux, de 375 $\mu$m, et une largeur supérieure Lmax de 625$\mu$m dans le plan du séparateur en contact avec la plaque de recouvrement. La plaque de recouvrement est percée pour réaliser les orifices d'entrée et de sortie puis collée thermiquement avec le séparateur afin de réaliser un assemblage étanche. Les canaux d'acheminement des orifices d'entrée 440a, 440b, 440c, présentent un angle de respectivement de 45°, 0° et moins 45° par rapport au canal de séparation 450 dans le plan du séparateur, de manière à ralentir le débit des fluides provenant des canaux d'acheminement présentant un angle de 45 ° ou - 45°, 440a, 440c, par rapport au canal de séparation. Le canal de séparation 450 rectiligne, présente une longueur de 80 mm, permettant, lors du fonctionnement du système, aux cellules, particules ou molécules de devenir acoustiquement concentrées dans le canal d'acheminement 470b puis 470d, dont les axes sont identiques à l'axe du canal de séparation.

Alternativement, le séparateur 422 du dispositif 400 comprend deux évidements (non représentés) réalisés le long du canal de séparation 450, et susceptibles de recevoir chacun un transducteur à ultrasons (non représenté). Ce mode de réalisation permet l'obtention d'une onde stationnaire dans le séparateur 422 si celui-ci réalisé dans un matériau peu réfléchissant aux ondes acoustiques, le matériau étant souple et fin tel que des matériaux plastiques, polymères, silicones. Un silicone préférentiellement utilisé est le Polydiméthylsiloxane (PDMS). Préférentiellement, chaque transducteur à ultrasons comprend une lame ¼ onde adaptatrice dont l'impédance acoustique est calculée pour minimiser les pertes d'énergie, et donc l'échauffement, entre le transducteur à ultrasons et les parois du dispositif.

Préférentiellement la plaque de recouvrement (non représentée) est réalisée en PDMS ou en matériau plastique moulé tel que du Polycarbonate (PC), poly(méthacrylate de méthyle) (PMMA), polypropylène (PP), Polystyrène (PS), Acrylonitrile butadiène styrène (ABS), Copolymère d'Oléfine Cyclique (COC), Polymère d'Oléfine Cyclique (COP), Polyoxyméthylène (POM).

Ce dispositif 400 est susceptible de mettre en œuvre des procédés de préparation d'échantillons biologiques par acoustophorèse tel que le procédé selon l'invention. Pour cela, tout ou partie de l'échantillon biologique à traiter, est introduit dans l'orifice d'entrée 430a du dispositif 400. Une solution tampon est introduite dans l'orifice d'entrée 430b, le tampon et l'échantillon étant introduits à des débits respectifs aptes à générer un flux laminaire dans le canal de séparation. Un transducteur ultrasonore, tel qu'un transducteur piézoélectrique, accolé au canal de séparation est alors activé par un signal de commande, de manière à réaliser une étape de séparation dudit échantillon biologique par acoustophorèse. Cette séparation permet de favoriser la concentration des particules non-spécifiques, tel que des débris alimentaires, présents dans l'échantillon, dans le canal d'acheminement 470b dudit dispositif d'acoustophorèse. L'échantillon décomplexifié est obtenu dans l'orifice de sortie 460a. Une seconde étape de séparation dudit échantillon biologique par acoustophorèse est réalisé sur la partie résultante à la première séparation dans le canal 470b. Cette séparation permet de favoriser la concentration des particules non-spécifiques, tel que des débris alimentaires, présents dans l'échantillon, dans l'orifice de sortie 460c dudit dispositif d'acoustophorèse. L'échantillon décomplexifié une seconde fois est obtenu dans l'orifice de sortie 460b. La collection et le mélange des échantillons décomplexifiés issus des orifices de sortie 460a et 460b permettent d'augmenter le rendement de collection des microorganismes présents dans l'échantillon biologique. En effet les microorganismes entraînés avec les particules non spécifiques lors de la première séparation dans le canal 470b sont susceptibles d'être séparés par acoustophorèse dans ce canal 470b et de pouvoir ainsi être collectés suite à cette seconde séparation dans l'orifice 460b.

La position relative et la longueur du canal de seconde séparation 470b vis-à-vis du canal de séparation principal 450 est choisie en fonction des applications, des performances et des débits souhaités.

Comme représenté sur la figure 6c, l'invention concerne également un quatrième mode de réalisation d'un dispositif 1300 comprenant deux parties fluidiques, un séparateur 1322 et une plaque de recouvrement (non représentée), ces deux parties étant sensiblement planes. Ces deux parties sont assemblées de façon hermétique. Des canaux sont réalisés dans le séparateur 1322. Le dispositif 1300 comprend deux orifices d'entrée 1330a, 1330b, en communication fluidique avec un premier canal de séparation 1350 par le biais de canaux d'acheminement respectivement 1340a, 1340c pour l'orifice 1330a et 1340b pour l'orifice 1330b. Le canal de séparation 1350 est en communication fluidique avec un orifice de sortie 1360b, par le biais d'un canal d'acheminement 1370b. Le canal de séparation 1350 est également en communication fluidique avec un second canal de séparation 1350' par le biais de deux canaux d'acheminements 1370a et 1370c. Le second canal de séparation 1350' est en communication fluidique avec un orifice de sortie 1360b',

par le biais d'un canal d'acheminement 1370b' et en communication fluidique avec un orifice de sortie 1360a, par le biais deux canaux d'acheminements 1370a' et 1370c'.

Le dispositif 1300 comprend également un orifice d'entrée 1330b', en communication fluidique avec le second canal de séparation 1350' via un canal d'acheminement 1340b'.

Le dispositif 1300 est agencé pour qu'un ou plusieurs transducteur(s) à ultrasons, non représenté(s), puisse être intégré dans ou accolé à une paroi desdits canaux de séparation 1350 et 1350'. Le(s) transducteur(s) à ultrasons ainsi intégré(s) ou accolé(s) est(sont) apte(s) à transmettre des oscillations mécaniques en ondes acoustiques multiples pouvant agir sur le contenu des canaux de séparation et y générer une onde acoustique stationnaire. Un générateur de courant alternatif associé à un amplificateur de signal, non représentés, peut être connecté électriquement au(x) transducteur(s) afin de générer un signal de commande dont la fréquence, la forme d'onde et l'amplitude sont connues.

Cette configuration permet d'améliorer la pureté de l'échantillon décomplexifié. En effet, après avoir concentré les particules non-spécifiques vers l'orifice de sortie 1360b, l'échantillon décomplexifié est injecté dans le second canal de séparation 1350' via les canaux 1370a et 1370c. L'échantillon décomplexifié est alors séparé une seconde fois des particules non-spécifiques pouvant être toujours présentes, celles-ci se retrouvant dans l'orifice 1360b'. L'échantillon décomplexifié une seconde fois est obtenu dans l'orifice de sortie 1360a.

Dans un mode de réalisation particulier du dispositif 1300, le séparateur 1322 est réalisé dans une plaquette de verre de 1mm d'épaisseur revêtue d'une couche de chrome et d'une couche de résine photosensible. Après développement de la couche photosensible, le verre est gravé par de l'acide fluorhydrique (HF). Les canaux d'acheminement 1340a et 1340c, 1370a, 1370c, 1370a' et 1370c' présentent une profondeur P de 125 $\mu$m, une largeur inférieure Lmin, au fond des canaux, de 300 $\mu$m, et d'une largeur supérieure Lmax de 550 $\mu$m dans le plan du séparateur en contact avec la plaque de recouvrement. Les canaux d'acheminement 1340b, 1340b', 1370b et 1370b' ainsi que les canaux de séparation 1350 et 1350' présentent une profondeur P de 125 $\mu$m, une largeur inférieure Lmin, au fond des canaux, de 375 $\mu$m, et une largeur supérieure Lmax de 625 $\mu$m dans le plan du séparateur en contact avec la plaque de recouvrement. La plaque de recouvrement est percée pour réaliser les orifices d'entrée et de sortie puis collée thermiquement avec le séparateur afin de réaliser un assemblage étanche.

Les canaux d'acheminement des orifices d'entrée 1340a, 1340b, présentent un angle de respectivement de 45°, 0° et moins 45° par rapport au canal de séparation 1350 dans le plan du séparateur, de manière à ralentir le débit des fluides provenant des canaux d'acheminement présentant un angle de 45 ° ou -45°, 1340a, 1340c, par rapport au canal de séparation 1350. Le canal de séparation 1350 rectiligne, présente une longueur de 19,5 mm, permettant aux cellules, particules ou molécules de devenir acoustiquement concentrés dans le canal d'acheminement 1370b lors du fonctionnement du système. L'axe du canal d'acheminement 1370b est identique à l'axe du canal de séparation.

Les canaux d'acheminement 1370a, 1370b, 1340b' présentent un angle de respectivement de 45°, 0° et moins 45° par rapport au canal de séparation 1350' dans le plan du séparateur, de manière à ralentir le débit des fluides provenant des canaux d'acheminement présentant un angle de 45 ° ou - 45°, 1340a, 1340c, par rapport au second canal de séparation 1350'. Le second canal de séparation 1350' rectiligne, présente une longueur de 19,5 mm, permettant aux cellules, particules ou molécules de devenir acoustiquement concentrés dans le canal d'acheminement 1370b' lors du fonctionnement du système. L'axe du canal d'acheminement 1370b' est identique à l'axe du canal de séparation 1350'. Les cellules, particules ou molécules sont acoustiquement concentrées dans le canal central 1370b', en fonction de leur densité, leur taille et leur compressibilité.

Alternativement, le séparateur 1322 du dispositif 1300 comprend quatre évidements (non représentés) réalisés le long des canaux de séparation 1350 et 1350', et susceptibles de recevoir chacun un transducteur à ultrasons (non représentés). Ce mode de réalisation permet l'obtention d'une onde stationnaire dans le séparateur 1322 si celui-ci est réalisé dans un matériau peu réfléchissant aux ondes acoustiques, le matériau étant souple et fin tel que des matériaux plastiques, polymères, silicones. Un silicone préférentiellement utilisé est le Polydiméthylsiloxane (PDMS). Préférentiellement, chaque transducteur à ultrasons comprend une lame ¼ onde adaptatrice dont l'impédance acoustique est calculée pour minimiser les pertes d'énergie, et donc l'échauffement, entre le transducteur à ultrasons et les parois du dispositif. Préférentiellement la plaque de recouvrement (non représentée) est réalisée en PDMS ou en matériau plastique moulé tel que du Polycarbonate (PC), poly(méthacrylate de méthyle) (PMMA), polypropylène (PP), Polystyrène (PS), Acrylonitrile butadiène styrène (ABS), Copolymère d'Oléfine Cyclique (COC), Polymère d'Oléfine Cyclique (COP), Polyoxyméthylène (POM).

Ce dispositif 1300 est susceptible de mettre en œuvre des procédés de préparation d'échantillons biologiques par acoustophorèse tel que le procédé selon l'invention. Pour cela, tout ou partie de l'échantillon biologique à traiter, est introduit dans l'orifice d'entrée 1330a du dispositif 1300. Une solution tampon est introduite dans les orifices d'entrée 1330b et 1330b', les tampons et l'échantillon étant introduits à des débits respectifs aptes à générer un flux laminaire dans les canaux de séparations 1350 et 1350'. Un ou des transducteur(s) ultrasonore(s), accolé(s) aux canaux de séparation est (sont) alors activé(s) par un signal de commande, de manière à réaliser deux étapes successives de séparation dudit échantillon biologique par acoustophorèse. Cette séparation permet de favoriser une première fois la concentration des particules non-spécifiques, tel que des débris alimentaires, présents dans l'échantillon, dans l'orifice de sortie 1360b. A la suite de cette première séparation, un seconde séparation permet de favoriser une seconde fois la concentration des particules non-spécifiques, tel que des débris alimentaires toujours présentes dans l'échantillon décomplexifié en provenance des canaux 1370a et 1370c dans l'orifice de sortie 1360b'. L'échantillon décomplexifié une seconde fois et présentant une pureté améliorée étant obtenu dans l'orifice de sortie 1360a.

La position relative et la longueur du canal de seconde séparation 1350' vis-à-vis du canal de séparation principal 1350' est choisie en fonction des applications, des performances et des débits souhaités. Avantageusement, deux solutions tampons de type ou de densité différentes sont introduites dans les orifices d'entrée 1330b et 1330b'.

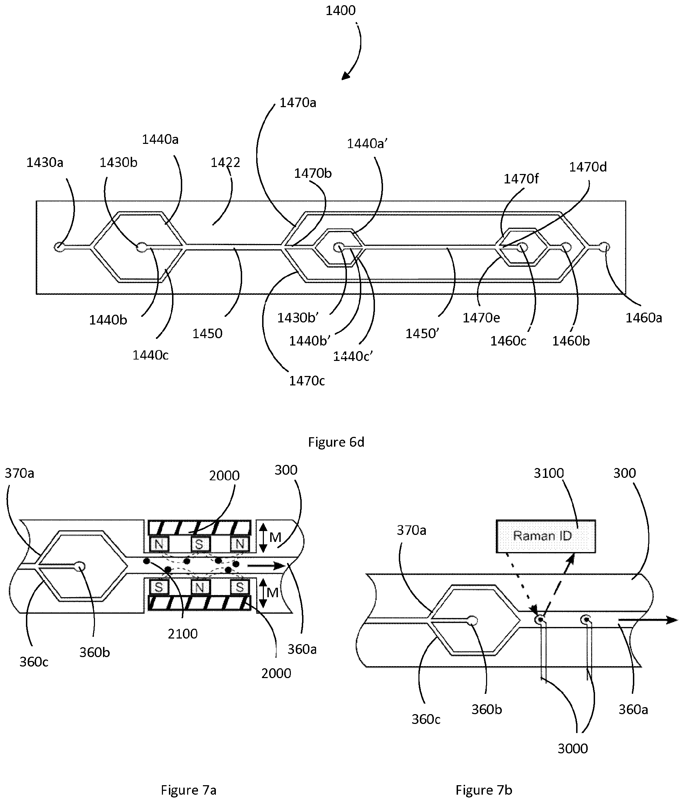

Comme représenté sur la figure 6d, l'invention concerne également un cinquième dispositif 1400 comprenant deux parties fluidiques, un séparateur 1422 et une plaque de recouvrement (non représentée), ces deux parties étant sensiblement planes. Ces deux parties sont assemblées de façon hermétique. Des canaux fluidiques sont réalisés dans le séparateur 1422. Le dispositif 1400 comprend deux orifices d'entrée 1430a, 1430b, en communication fluidique avec un premier canal de séparation 1450 par le biais de canaux d'acheminement respectivement 1440a, 1440c, pour l'orifice 1430a et 1440b pour l'orifice 1430b.

Le premier canal de séparation 1450 est également en communication fluidique avec un orifice de sortie 1460a, par le biais de canaux d'acheminement 1470a et 1470c.

Le premier canal de séparation 1450 est enfin en communication fluidique avec un second canal de séparation 1450', par le biais de canaux d'acheminement 1440a' et 1440c'.

Le dispositif 1400 comprend également un orifice d'entrée 1430b', en communication fluidique avec le second canal de séparation 1450' par le canal d'acheminement 1440b'.

Le second canal de séparation 1450' est également en communication fluidique avec deux orifices de sortie 1460b, 1460c, par le biais de canaux d'acheminement, respectivement 1470d pour l'orifice 1460c ; 1470e et 1470f pour l'orifice de sortie 1460b.

Le dispositif 1400 est agencé pour qu'un ou plusieurs transducteur(s) à ultrasons, non représenté(s), puisse être intégré(s) dans ou accolé(s) à une paroi desdits canaux de séparation 1450 et 1450'. Le(s) transducteur(s) à ultrasons ainsi intégré(s) ou accolé(s) est(sont) apte(s) à transmettre des oscillations mécaniques en ondes acoustiques multiples pouvant agir sur le contenu des canaux de séparation et y générer une onde acoustique stationnaire. Un générateur de courant alternatif associé à un amplificateur de signal, non représentés, peut être connecté électriquement au(x) transducteur(s) afin de générer un signal de commande dont la fréquence, la forme d'onde et l'amplitude sont connues.

Dans un mode de réalisation particulier du dispositif 1400, le séparateur 1422 est réalisé dans une plaquette de verre de 1mm d'épaisseur revêtue d'une couche de chrome et d'une couche de résine photosensible. Après développement de la couche photosensible, le verre est gravé par de l'acide fluorhydrique (HF). Les canaux d'acheminement 1440a, 1440c, 1470a, 1470c, 1470e et 1470f présentent une profondeur P de 125 $\mu$m, une largeur inférieure Lmin, au fond des canaux, de 300 $\mu$m, et une largeur supérieure Lmax de 550 $\mu$m dans le plan du séparateur en contact avec la plaque de recouvrement. Les canaux d'acheminement 1440b, 1470b et 1470d ainsi que les canaux de séparation 1450 et 1450' présentent une profondeur P de 125 $\mu$m, une largeur inférieure Lmin, au fond des canaux, de 375 $\mu$m, et une largeur supérieure Lmax de 625$\mu$m dans le plan du séparateur en contact avec la plaque de recouvrement. La plaque de recouvrement est percée pour réaliser les orifices d'entrée et de sortie puis collée thermiquement avec le séparateur afin de réaliser un assemblage étanche. Les canaux d'acheminement 1440a, 1440c, présentent un angle de respectivement de 45° et moins 45° par rapport au canal de séparation 1450 dans le plan du séparateur, de manière à ralentir le débit des fluides provenant de ces canaux d'acheminement. De la même manière, les canaux d'acheminement 1440a', 1440c', présentent un angle de respectivement de 45° et moins 45° par rapport au canal de séparation 1450' dans le plan du séparateur.

Les canaux de séparation 1450 et 1450' rectilignes, présente une longueur de 22,5 mm, permettant, lors du fonctionnement du système, aux cellules, particules ou molécules de devenir acoustiquement concentrées dans le canal d'acheminement 1470b puis 1470d, dont les axes sont identiques aux axes des canaux de séparation.

Alternativement, le séparateur 1422 du dispositif 1400 comprend quatre évidements (non représentés) réalisés le long des canaux de séparation 1450 et 1450', et susceptibles de recevoir chacun un transducteur à ultrasons (non représentés). Ce mode de réalisation permet l'obtention d'une onde stationnaire dans le séparateur 1422 si celui-ci réalisé dans un matériau peu réfléchissant aux ondes acoustiques, le matériau étant souple et fin tel que des matériaux plastiques, polymères, silicones. Un silicone préférentiellement utilisé est le Polydiméthylsiloxane (PDMS). Préférentiellement, chaque transducteur à ultrasons comprend une lame ¼ onde adaptatrice dont l'impédance acoustique est calculée pour minimiser les pertes d'énergie, et donc l'échauffement, entre le transducteur à ultrasons et les parois du dispositif. Préférentiellement la plaque de recouvrement (non représentée) est réalisée en PDMS ou en matériau plastique moulé tel que du Polycarbonate (PC), poly(méthacrylate de méthyle) (PMMA), polypropylène (PP), Polystyrène (PS), Acrylonitrile butadiène styrène (ABS), Copolymère d'Oléfine Cyclique (COC), Polymère d'Oléfine Cyclique (COP), Polyoxyméthylène (POM).

Ce dispositif 1400 est susceptible de mettre en œuvre des procédés de préparation d'échantillons biologiques par acoustophorèse tel que le procédé selon l'invention. Pour cela, tout ou partie de l'échantillon biologique à traiter, est introduit dans l'orifice d'entrée 1430a du dispositif 1400. Une solution tampon est introduite dans l'orifice d'entrée 1430b, le tampon et l'échantillon étant introduits à des débits respectifs aptes à générer un flux laminaire dans le canal de séparation 1450. Un transducteur ultrasonore, tel qu'un transducteur piézoélectrique, accolé au canal de séparation 1450 est alors activé par un signal de commande, de manière à réaliser une étape de séparation dudit échantillon biologique par acoustophorèse. Cette séparation permet de favoriser la concentration des particules non-spécifiques, tel que des débris alimentaires, présents dans l'échantillon, dans le canal d'acheminement 1470b dudit dispositif d'acoustophorèse. L'échantillon décomplexifié est obtenu dans l'orifice de sortie 1460a. Une seconde étape de séparation dudit échantillon concentré par acoustophorèse est également réalisée sur la partie résultante à la première séparation dans le canal 1450'. Pour cela un second tampon propre est introduit simultanément dans l'orifice d'entrée 1430b' afin d'extraire les espèces d'intérêt résiduelle toujours présente dans l'échantillon concentré. Cette séparation permet de favoriser la concentration des particules non-spécifiques, tel que des débris alimentaires, toujours présents dans l'échantillon concentré, dans l'orifice de sortie 1460c dudit dispositif d'acoustophorèse. L'échantillon concentré et alors décomplexifié une seconde fois puis obtenu dans l'orifice de sortie 1460b. La collection et le mélange des échantillons décomplexifiés issus des orifices de sortie 1460a et 1460b permettent d'augmenter le rendement de collection des microorganismes présents initialement dans l'échantillon biologique. En effet les microorganismes entraînés avec les particules non spécifiques lors de la première séparation vers le canal 1450' via les canaux 1440a' et 1440c' sont susceptibles d'être séparés par acoustophorèse dans ce canal 1450' et de pouvoir ainsi être collectés suite à cette seconde séparation dans l'orifice 1460b. Le rendement d'extraction des microorganismes et ainsi amélioré.

La position relative et la longueur du canal de seconde séparation 1450' vis-à-vis du canal de séparation principal 1450' est choisie en fonction des applications, des performances et des débits souhaités. Avantageusement, deux solutions tampons de type ou de densité différentes sont introduites dans les orifices d'entrée 1430b et 1430b'.

Quel que soit le mode de réalisation du dispositif permettant la mise en œuvre de la méthode selon l'invention, le dispositif peut comprendre, autour du canal d'acheminement vers un orifice de sortie contenant l'échantillon décomplexifié, des supports d'aimants mobiles permettant la capture d'échantillon mise en présence de particules magnétiques tel que de la silice magnétique. Comme représenté sur la figure 7a en sortie du dispositif 300, à proximité de l'orifice 360a, deux supports d'aimants mobiles 2000 sont disposées. Les aimants ainsi disposés de part et d'autres du canal d'acheminement vers l'orifice de sortie 360a permettent de capturer en flux continu des microorganismes par déplacement de particules de silice magnétiques 2100 de manière perpendiculaire au flux de l'échantillon décomplexifié. Le mouvement M des supports d'aimant s'effectue par exemple perpendiculairement au canal et de façon alternative entre les deux supports afin de favoriser le mélange de l'échantillon et des particules.

Quel que soit le mode de réalisation du dispositif permettant la mise en œuvre de la méthode selon l'invention, le dispositif peut comprendre, autour du canal d'acheminement vers un orifice de sortie contenant l'échantillon décomplexifié, des électrodes de Di ElectroPhorese (DEP) déposées sur la surface du dispositif au niveau de l'orifice de sortie. Comme représenté sur la figure 7b en sortie du dispositif 300, à proximité de l'orifice 360a, deux électrodes de Di ElectroPhorese (DEP) 3000 sont disposées afin de permettent la réalisation d'une étape de capture spécifique. Ces électrodes peuvent être soient fonctionnalisées avec des antigènes de captures dans le cas d'une capture sur un panel d'espèces recherchées, ou non fonctionnalisées utilisant alors uniquement les propriétés bien connues de capture par la charge de la technique DEP. L'identification des espèce(s) d'intérêt capturée(s) peut être dans les deux cas de figure confirmée ou effectuée par exemple par identification Raman 3100.

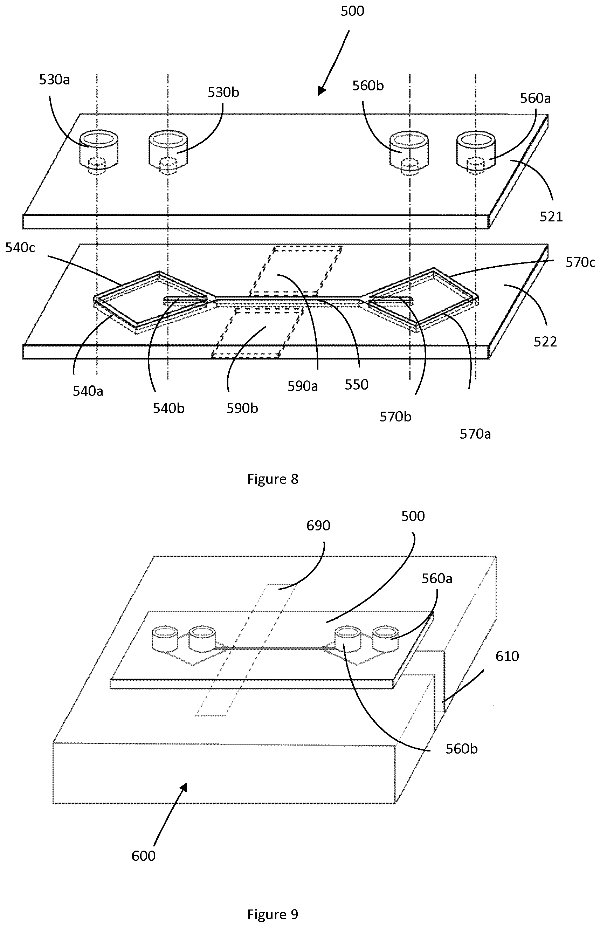

La figure 8, représente un sixième dispositif 500 permettant la mise en œuvre de la méthode selon l'invention et comprenant deux parties fluidiques, un séparateur 522 et une plaque de recouvrement 521, ces deux parties étant sensiblement planes. Ces deux parties sont assemblées de façon hermétique. Des canaux fluidiques sont réalisés dans le séparateur 522. Le dispositif 500 comprend deux orifices d'entrée 530a, 530b, réalisés dans la plaque de recouvrement 521, en communication fluidique avec au moins un canal de séparation 550 par le biais de canaux d'acheminement respectivement 540a, 540c, pour l'orifice 530a et 540b pour l'orifice 530b. Le canal de séparation 550 est également en communication fluidique avec deux orifices de sortie 560a, 560b, par le biais de canaux d'acheminement, respectivement 570a et 570c pour l'orifice 560a, 570b pour l'orifice 560b. Les orifices d'entrée et de sortie forment des réservoirs de manière à pouvoir réaliser des opérations de dépose d'un échantillon à décomplexifier par pipetage et de la solution tampon ainsi que des opérations de connexion à un système de mise en pression ou vide asservi et régulé sur la mesure de débit et permettant de piloter de manière reproductible l'introduction et l'écoulement des échantillons biologique et du tampon dans le canal de séparation. Le dispositif 500 est agencé pour qu'un transducteur à ultrasons, non représenté, puisse être intégré dans ou accolé à une paroi dudit canal de séparation 550. Le transducteur à ultrasons ainsi intégré ou accolé est apte à transmettre des oscillations mécaniques en ondes acoustiques multiples pouvant agir sur le contenu du canal de séparation et y générer une onde acoustique stationnaire. Un générateur de courant alternatif non représenté peut être connecté électriquement au transducteur afin de générer un signal de commande du transducteur dont la fréquence, la forme d'onde et l'amplitude sont connues.

Dans un mode de réalisation particulier du dispositif 500, Le séparateur 522 est réalisé dans une plaquette de verre de 1mm d'épaisseur revêtue d'une couche de chrome et d'une couche de résine photosensible. Après développement de la couche photosensible, le verre est gravé par de l'acide fluorhydrique (HF). Les canaux d'acheminement 540a et 540c, 570a, 570c, présentent une profondeur P de 125 $\mu$m, une largeur inférieure Lmin, au fond des canaux, de 300 $\mu$m, et une largeur supérieure Lmax de 550 $\mu$m dans le plan du séparateur en contact avec la plaque de recouvrement. Les canaux d'acheminement 540b, 570b ainsi que le canal de séparation 550 présentent une profondeur P de 125 $\mu$m, une largeur inférieure Lmin, au fond des canaux, de 375 $\mu$m, et une largeur supérieure Lmax de 625$\mu$m dans le plan du séparateur en contact avec la plaque de recouvrement. La plaque de recouvrement est percée pour réaliser les orifices d'entrée et de sortie puis collée thermiquement avec le séparateur afin de réaliser un assemblage étanche. Les canaux d'acheminement des orifices d'entrée (540a, 540b, 540c) présentent un angle de respectivement de 45°, 0° et moins 45° par rapport au canal de séparation 550 dans le plan du séparateur, de manière à ralentir le débit des fluides provenant des canaux d'acheminement présentant un angle de 45° ou -45°, 540a, 540c, par rapport au canal de séparation. Le canal de séparation 550 rectiligne, présente une longueur de 80 mm, permettant aux cellules, particules ou molécules de devenir acoustiquement concentrés dans le canal d'acheminement 570b lors du fonctionnement du système. L'axe du canal d'acheminement 570b est identique à l'axe du canal de séparation 550.

Alternativement, le séparateur 522 du dispositif 500 comprend deux évidements, 590a, 590b, réalisés le long du canal de séparation 550, et susceptibles de recevoir chacun un transducteur à ultrasons (non représentés). Ce mode de réalisation permet l'obtention d'une onde stationnaire dans le séparateur 522 si celui-ci réalisé dans un matériau peu réfléchissants aux ondes acoustiques, le matériau étant souple et fin tel que des matériaux plastiques, polymères, silicones. Un silicone préférentiellement utilisé est le Polydiméthylsiloxane (PDMS). Préférentiellement, chaque transducteur à ultrasons comprend une lame ¼ onde adaptatrice dont l'impédance acoustique est calculée pour minimiser les pertes d'énergie, et donc l'échauffement, entre le transducteur à ultrasons et les parois du dispositif.

Préférentiellement la plaque de recouvrement (non représentée) est réalisée en PDMS ou en matériau plastique moulé tel que du Polycarbonate (PC), poly(méthacrylate de méthyle) (PMMA), polypropylène (PP), PolyStyrene (PS), Acrylonitrile butadiène styrène (ABS), Copolymère d'Oléfine Cyclique (COC), Polymère d'Oléfine Cyclique (COP), Polyoxyméthylène (POM).

Ce dispositif 500 est susceptible de mettre en œuvre des procédés de préparation d'échantillons biologiques par acoustophorèse tel que le procédé selon l'invention. Pour cela, tout ou partie de l'échantillon biologique à traiter est introduit dans l'orifice d'entrée 530a du dispositif 500. Une solution tampon est introduite dans l'orifice d'entrée 530b, le tampon et l'échantillon étant introduits à des débits respectifs aptes à générer un flux laminaire dans le canal de séparation. Un transducteur ultrasonore, tel qu'un transducteur piézoélectrique, accolé au canal de séparation est alors activé par un signal de commande, de manière à réaliser une étape de séparation dudit échantillon biologique par acoustophorèse. Cette séparation permet de favoriser la concentration des particules non-spécifiques, tel que des débris alimentaires, présents dans l'échantillon, dans l'orifice de sortie 560b dudit dispositif d'acoustophorèse. L'échantillon décomplexifié étant obtenu dans l'orifice de sortie 560a.

La figure 9 présente un support 600 pour dispositifs avec un dispositif 500 disposé et maintenu sur le support. Ce support permet par exemple de traiter un même échantillon biologique simultanément sur plusieurs dispositifs ou plusieurs échantillons différent simultanément en connectant un ou des réservoir(s) contenant l'(les) échantillon(s) tel qu'une seringue aux différents orifices d'entrée des dispositifs disposés sur le support. Alternativement, le support peut comprendre un capot articulé, non représenté, permettant de connecter directement les orifices d'entrée et de sortie du dispositif à des moyens de mise en pression et/ou vide, à la fermeture du capot. Ce support comprend un ou plusieurs socles de réception (non représentés) de dispositifs selon l'invention, permettant de placer et maintenir lesdits dispositifs. Le maintien des dispositifs peut être réalisé mécaniquement ou par mise en aspiration des dispositifs sur le(s) socle(s) de réception. Des transducteurs ultrasonores 690 peuvent être disposés dans ces socles de manière à pouvoir créer un onde stationnaire dans chacun des dispositifs disposés sur le support. Le transducteur étant accolé au dispositif quand celui-ci est disposé dans ledit socle. Alternativement un seul transducteur ultrasonore recouvrant plusieurs dispositif peut être disposé de manière à pouvoir créer un onde stationnaire dans chacun des dispositifs disposés sur le support.

Un espace de réception 610 permet notamment d'accoler un système de chauffage (non représenté) à au moins un des orifices de sortie du dispositif disposé sur le support. Ledit système de chauffage permettant d'incuber l'échantillon biologique traité et contenu dans au moins un des orifices de sortie 560a, 560b. De plus, l'espace de réception 610 peut comprendre un moyen de mesure de la densité optique de l'échantillon contenu dans au moins un des orifice de sortie 560a, de manière à éviter des manipulations supplémentaires pour réaliser cette opération. D'autre part, le support peut être disposé directement sur un agitateur ou comprendre un moyen d'agitation de manière à réaliser une étape d'agitation de l'échantillon biologique contenu dans un des orifices de sortie, avant, pendant ou suite à une étape d'incubation.

Ce support 600 permet également de disposer et maintenir différents dispositifs 200, 300, 400, 500, 800, selon l'invention, l'espace de réception 610 pouvant être facilement adapté par l'homme du métier.

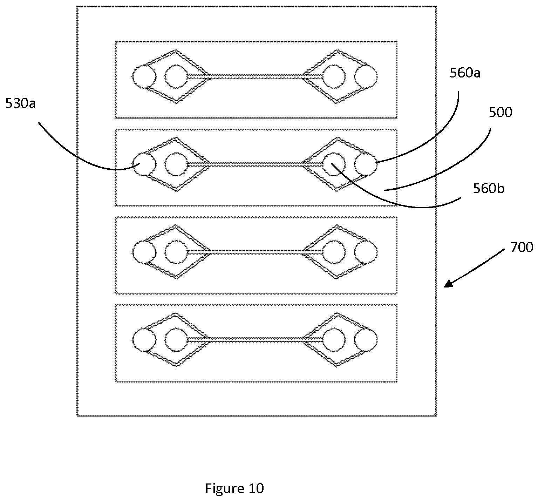

La figure 10 présente un support 700 pour dispositifs permettant la mise en œuvre de la méthode selon l'invention représenté avec quatre dispositifs 500 disposés et maintenus sur le support. Ce support permet par exemple de traiter un même échantillon biologique simultanément sur plusieurs dispositifs en connectant un réservoir contenant l'échantillon biologique tel qu'une seringue aux différents orifices d'entrée 530a des dispositifs disposés sur le support. Alternativement, le support permet de disposer les dispositifs de manière à présenter chacun des orifices d'entrée et de sortie alignés sur un même axe et selon un écartement défini. Cet écartement peut avantageusement être équivalent ou multiple à celui du pas d'une microplaque connue de l'homme du métier. De cette manière, les opérations de pipetage, automatiques ou manuelles, peuvent être réalisées directement dans plusieurs dispositifs simultanément, notamment à l'aide d'une pipette ou un distributeur à microplaques. Ce support comprend plusieurs socles de réception (non représentés) de dispositifs selon l'invention, permettant de placer et maintenir lesdits dispositifs. Des transducteurs ultrasonores (non représentés), ou un transducteur commun à plusieurs dispositifs, peuvent être disposés dans ces socles de manière à pouvoir créer un onde stationnaire dans chacun des dispositifs disposés sur le support. Le transducteur étant accolé au dispositif quand celui-ci est disposé dans ledit socle. De façon identique au support 600, le support 700 peut comprendre plusieurs espaces de réception (non représentés) permettant notamment d'accoler à au moins un des orifices de sortie de chacun des dispositifs disposés sur le support, un système de chauffage (non représenté) permettant d'incuber l'échantillon biologique traité et contenu dans au moins un des orifices de sortie 560a, 560b. De plus, chacun des espaces de réception peut comprendre un moyen de mesure de la densité optique de l'échantillon contenu dans au moins un des orifices de sortie 560a, de manière à éviter des manipulations supplémentaires pour réaliser cette opération. D'autre part, le support 700 peut être disposé directement sur un agitateur ou comprendre un moyen d'agitation de manière à réaliser une étape d'agitation de l'échantillon biologique contenu dans un des orifices de sortie, avant, pendant ou suite à une étape d'incubation. Ce support 700 permet également de disposer parallèlement plusieurs des différents dispositifs 200, 300, 400, 500, 800, selon l'invention, le nombre de socles de réception pouvant être facilement adapté par l'homme du métier. Les différents dispositifs disposés sur le support 700 peuvent être de même type ou de types différents.

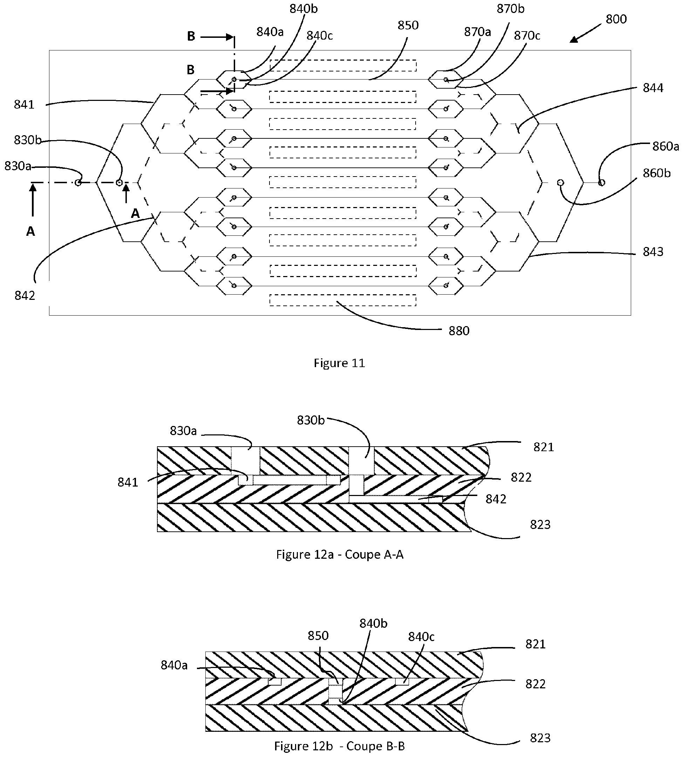

Les figures 11, 12a et 12b, représentent un dispositif multiplexe 800 permettant la mise en œuvre de la méthode selon l'invention et comprenant trois parties fluidiques, un séparateur 822, une plaque de recouvrement 821 et un socle 823, ces trois parties étant sensiblement planes. Ces trois parties sont assemblées de façon hermétique. Des canaux fluidiques sont réalisés dans le séparateur 822. Le dispositif 800 comprend deux orifices d'entrée 830a, 830b, en communication fluidique avec huit canaux de séparation 850, par le biais d'un réseau de canaux introduction 841, 842, et de canaux d'acheminement. Ainsi, les canaux de séparation 850 communiquent avec l'orifice 830a via le réseau de canaux d'introduction 841 ainsi que par les canaux d'acheminement 840a, 840c. De la même manière, les canaux de séparation 850 communiquent avec l'orifice 830b via le réseau de canaux d'introduction 842 ainsi que par le canal d'acheminement 840b. Les canaux de séparation 850 sont également en communication fluidique avec deux orifices de sortie 860a, 860b, par le biais d'un réseau de canaux d'aspiration 843, 844 et de canaux d'acheminement. Ainsi, les canaux de séparation 850 communiquent avec l'orifice 860a via le réseau de canaux d'aspiration 843 ainsi que par les canaux d'acheminement, 870a, 870c.De la même manière, les canaux de séparation 850 communiquent avec l'orifice 860b via le réseau de canaux d'aspiration 844 ainsi que par le canal d'acheminement 870b. Huit canaux de séparation 850 sont représentés, chacun des canaux 850 et des canaux d'acheminement associés formant un motif fluidique répété huit fois.

Le dispositif 800 est agencé pour qu'un transducteur à ultrasons, non représenté, puisse être intégré dans ou accolé à une paroi desdits canaux de séparation 850. Le transducteur à ultrasons ainsi intégré ou accolé est apte à transmettre des oscillations mécaniques en ondes acoustiques multiples pouvant agir sur le contenu de chaque canal de séparation et y générer une onde acoustique stationnaire. Un générateur de courant alternatif non représenté peut être connecté électriquement au transducteur afin de générer un signal de commande du transducteur dont la fréquence, la forme d'onde et l'amplitude sont connues. Alternativement, des ouvertures 880 traversantes ou des gravures d'une profondeur supérieure ou égale à celle des canaux de séparation sont effectuées dans le séparateur. Ces ouvertures ou gravures sont réparties de chaque côté des canaux de séparations 850. Ces ouvertures 880 permettent de mieux séparer acoustiquement les résonances de chaque canal de séparation.

Les réseaux de canaux d'introduction 841, 842 permettent de diviser le flux, par exemple d'échantillon biologique ou de tampon introduit dans les orifices d'entrée 830a et 830b. Cette parallélisation des canaux de séparation permet de traiter un volume d'échantillon par acoustophorèse à des débits plus important que sur un dispositif classique sans dégrader les performances d'extraction par l'augmentation du débit d'échantillon (en μL/mn). Ainsi si les réseaux divisent par deux le flux d'échantillon introduit, acheminant l'échantillon vers deux canaux de séparation, un débit deux fois supérieur peut être atteint pour un même taux de décomplexification. De la même manière, les réseaux d'aspiration 843, 844, permettent de collecter les échantillons ou tampon traités dans un seul orifice de sortie. Ces réseaux permettent également d'assurer un équilibre de pression en aval de l'étape de séparation par acoustophorèse.

Ce dispositif 800 est susceptible de mettre en œuvre des procédés de préparation d'échantillons biologiques par acoustophorèse tel que le procédé selon l'invention. Pour cela, tout ou partie de l'échantillon biologique à traiter, est introduit dans l'orifice d'entrée 830a du dispositif 800. Une solution tampon est introduite dans l'orifice d'entrée 830b, le tampon et l'échantillon étant introduits à des débits respectifs aptes à générer un flux laminaire dans les canaux de séparation. Un transducteur ultrasonore, tel qu'un transducteur piézoélectrique, accolé aux canaux de séparations est alors activé par un signal de commande, de manière à réaliser une étape de séparation dudit échantillon biologique par acoustophorèse. Cette séparation permet de favoriser la concentration des particules non-spécifiques, tel que des débris alimentaires, présents dans l'échantillon, dans l'orifice de sortie 860b. L'échantillon décomplexifié étant obtenu dans l'orifice de sortie 860a. Ce dispositif présentant huit canaux de séparations, un débit de traitement d'échantillon biologique huit fois supérieur à un dispositif de l'état de l'art peut être atteint sur un même dispositif. De la même manière, un volume d'échantillon (en mL) huit fois plus important peut être traité dans un temps identique sans modification de débit (μL/min) conservant ainsi les performances d'extraction intactes. De façon plus générale, ce cinquième dispositif multiplexe comprend deux orifices d'entrée en communication fluidique avec au moins deux canaux de séparation, par le biais d'un réseau de canaux introduction et de canaux d'acheminement. Lesdits canaux de séparation communiquent également avec deux orifices de sortie par le biais d'un réseau de canaux d'aspiration et de canaux d'acheminement. Ce motif de réalisation associé à chacun des canaux de séparation permet d'adapter le débit de traitement de l'échantillon en fonction de l'application souhaitée, en multipliant le nombre de canaux de séparation parallélisés et de canaux d'acheminement associés.

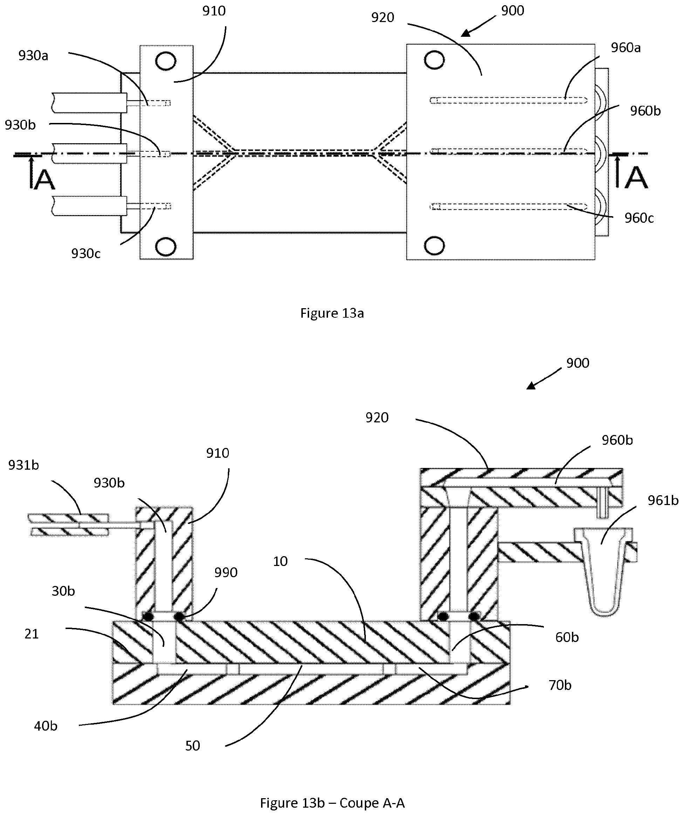

Comme représenté sur les figures 13a et 13b, l'invention concerne également un dispositif de connexion 900 permettant de connecter des orifices d'un dispositif microfluidique à un moyen d'introduction ou d'aspiration. Ce dispositif est représenté connecté à un dispositif 10 de l'état de l'art.

Le dispositif de connexion 900 comprend un connecteur d'entrées 910, et un connecteur de sorties 920. Le connecteur d'entrées 910 comprend trois canaux d'acheminement 930a, 930b, 930c aptes à coopérer avec les orifices d'entrée du dispositif 10, respectivement 30a, 30b, 30c. Le connecteur d'entrée 910 permet ainsi de venir connecter des moyens d'introduction d'échantillon biologique ou de solution tampon sans manipuler directement le dispositif microfluidique 10. Par exemple, un tube d'introduction d'une seringue 931b, visible sur la figure 13b, est connecté au canal d'acheminement 930b afin de pouvoir introduire une solution dans l'orifice 30b. Un arrangement similaire est réalisé en regard des orifices d'entrée 30a et 30c afin de pouvoir introduire l'échantillon biologique dans les orifices d'entrée 30a et 30c.

Le connecteur de sorties 920 comprend trois canaux d'acheminement 960a, 960b, 960c aptes à coopérer avec les orifices de sortie du dispositif 10, respectivement 60a, 60b, 60c. Le connecteur de sorties 920 permet ainsi de venir connecter des moyens d'aspiration ou des moyens de collecte d'échantillon biologique ou de solution tampon sans manipuler directement le dispositif microfluidique 10 et sans nécessiter un moyen d'aspiration ou de pompage connecté en sortie du dispositif microfluidique. Par exemple, un tube de collecte 961b visible sur la figure 13b, tel qu'un tube « Eppendorf », est disposé en regard du canal d'acheminement 960b afin de pouvoir collecter la solution en provenance de l'orifice de sortie 60b. Un arrangement similaire est réalisé en regard des orifices de sortie 60a et 60c afin de collecter l'échantillon décomplexifié directement dans des tubes de collecte.

Le dispositif 10 est disposé entre les connecteurs 910 et 920 et un support, non représenté. Les connecteurs 910 et 920 sont maintenus sur ledit support par tout moyen, notamment par vissage , par montage en force ou par des ressorts de rappels, de manière à assurer une connexion hermétique avec les orifices d'entrée et de sortie du dispositif. Avantageusement, les connecteurs comprennent des joints toriques 990 afin de garantir cette étanchéité quand le connecteur est maintenu.

Bien entendu, la géométrie de ce dispositif de connexion 900, des connecteurs 910 et 920 et le nombre de canaux d'acheminement des connecteurs pourront aisément être adaptés pour être utilisé avec un ou des dispositifs 200, 300, 400, 500, 800, selon l'invention.

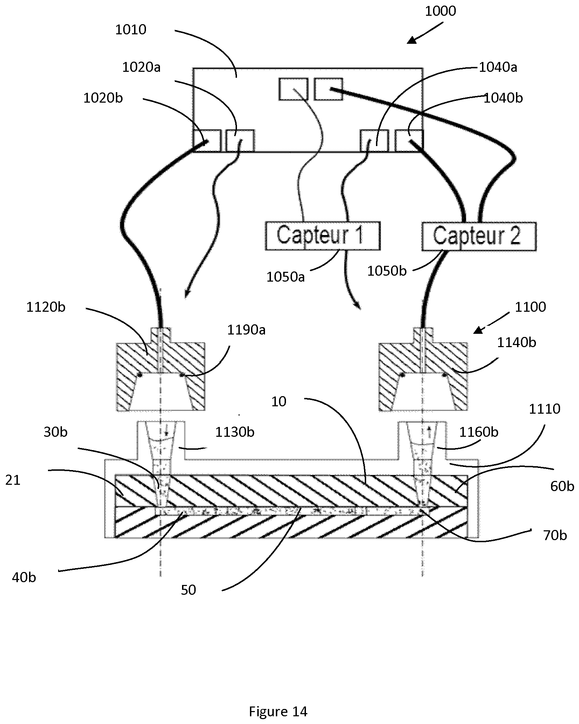

La figure 14 représente un système de régulation d'air 1000 et des dispositifs de connexion 1100 associés permettant la réalisation de la méthode selon l'invention par asservissement de l'introduction et de l'aspiration des liquides introduits. Le système est ici représenté avec un dispositif 10 de l'état de l'art représenté ici selon la coupe A-A de la figure 1.

Le système de régulation 1000 comprend un générateur de pression ou de vide 1010. Le générateur 1010 comprend deux sorties de pression 1020a et 1020b susceptibles de délivrer la pression d'air nécessaire à l'introduction de l'échantillon biologique ou de la solution tampon d'une méthode selon l'invention à un débit régulé dans un dispositif microfluidique. Le générateur 1010 comprend également deux entrées de vide 1040a et 1040b susceptibles de générer un différentiel de pression d'air suffisant pour aspirer l'échantillon biologique et la solution tampon d'une méthode selon l'invention à un débit régulé dans un dispositif microfluidique. A ces fins, les entrées de vide 1040a et 1040b sont connectées au dispositif via deux capteurs de débits d'air 1050a et 1050b afin de mesurer et d'asservir la régulation de pression d'air introduit ou le vide d'aspiration de l'échantillon et de la solution tampon d'une méthode selon l'invention. Cette régulation permet ainsi d'obtenir un débit constant de séparation et de décomplexification de l'échantillon biologique en limitant les risques de perte de charge.

Dans ce but, le générateur est connecté aux orifices d'entrée et de sortie du dispositif par l'intermédiaire du dispositif de connexion 1100. Ce dispositif comprend un connecteur d'entrée par orifice d'entrée ainsi qu'un connecteur de sortie par orifice de sortie. Seul les connecteurs d'entrée 1120b et 1140b sont représentés par souci de clarté de la figure.

Le dispositif de connexion 1100 comprend également un capot 1110 disposé et maintenu sur le dispositif 10. Le capot 1110 comprend un réservoir en regard de chacun des orifices d'entrée et de sortie du dispositif 10. Seuls le réservoir 1130b, coopérant avec l'orifice 30b et le réservoir 1160b coopérant avec l'orifice 60b sont représentés par souci de clarté de la figure. Le capot 10 peut être réalisé en matière plastique, par exemple par injection et permet de capoter un dispositif afin de présenter en regard de chaque orifice d'entrée et de sortie du dispositif maintenu des réservoirs. Ces réservoirs peuvent notamment être utilisés pour la dépose et la collecte d'échantillons et de solutions tampons. Le capot est maintenu sur un support, non représenté, de manière à réaliser une connexion hermétique avec les orifices du dispositif 10. Le capot peut être maintenu sur le support par tout moyen, notamment par vissage, par montage en force ou par la pression d'un ressort de rappel.

Un connecteur d'entrée 1120b est apte à être disposé et maintenu sur le capot 1110 de manière à coopérer avec le réservoir 1130b dudit capot. Un connecteur de sortie 1140b est également apte à être disposé et maintenu sur le capot 1110 de manière à coopérer avec le réservoir 1160b dudit capot. Les connecteurs présentent avantageusement des joints toriques d'étanchéité et sont maintenus sur le capot par des vis ou des ressorts. Les connecteurs peuvent également être maintenus sur le capot par tout moyen, notamment par vissage, par montage en force ou par la pression d'un ressort de rappel.

Bien entendu, la géométrie de ce dispositif de connexion 1100 et notamment des connecteurs pourra aisément être adaptée pour être utilisé avec un dispositif 200, 300, 400, 500, 800, permettant la mise en œuvre de la méthode selon l'invention.

Les exemples ci-après permettront de mieux appréhender la présente invention. Toutefois, ces exemples ne sont donnés qu'à titre illustratif et ne doivent en aucun cas être regardés comme limitant la portée de ladite invention d'une quelconque manière.

**Exemple : traitement d'échantillons alimentaires**

**A) Assemblage d'un dispositif permettant de mettre en œuvre le procédé selon l'invention**

Un dispositif microfluidique est réalisé conformément au dispositif représenté sur la figure 1.

Comme illustrés sur la figure 3, les trois orifices d'entrées 30a, 30b, 30c, et les trois orifices de sorties 60a, 60b, 60c, sont connectés à des moyens d'introduction, tel que des seringues (Becton Dickinson Plastipak TM, Espagne) via un assemblage comprenant un embout de pipette (Eppendorf, Royaume-Uni) connecté à un tube de Tygon (0,03 en ID, 0,09 en OD, Cole-Parmer, Royaume-Uni). Un embout de pipette est collé hermétiquement à chacun des orifices, un tube de tygon est alors inséré puis collé dans l'embout de pipette et enfin assemblé avec l'embout de la seringue. De cette manière, trois seringues d'introduction 130a, 130b, 130c, permettent d'introduire un fluide dans chacun des orifices d'entrée 30a, 30b, 30c, tandis que trois moyens d'aspiration tel que des seringues 140a, 140b, 140c, permettent d'aspirer le fluide dans chacun des orifices de sortie 60a, 60b, 60c. Alternativement, un dispositif de connexion 900 peut être utilisé pour connecter chaque seringue au dispositif.

Les trois seringues d'aspiration 140a, 140b, 140c, permettent d'assurer un équilibre de pression, et de débit, dans les trois canaux d'acheminement aux orifices de sorties, en aval du canal de séparation. De cette manière, le dispositif est moins sujet à d'éventuels bouchage pouvant être dus à des particules de taille importante entravant la circulation du fluide dans un des canaux.

Des pompes à seringues (Harvard Apparatus, Royaume-Uni), non représentées, sont utilisés pour contrôler les débits d'introduction des échantillons et tampon.

Un transducteur piézo-électrique d'une dimension de 10mm x 30 mm en céramique (Pz26, Ferroperm Piezoceramics, USA) avec une plage de fréquences de résonance de 1 MHz et 2 MHz est fixé au dispositif 10 par un gel à ultrasons (Anagel™ Royaume-Uni). Ce transducteur permet de générer des ultrasons utilisés pour induire une onde stationnaire entre les parois du canal de séparation. Un mouvement des particules induit par le phénomène d'acoustophorèse peut ainsi être obtenu par l'application d'un courant alternatif (AC) au transducteur. Ce signal de commande est introduit dans le circuit par un générateur de fonction (Agilent, modèle 33210A, 10 MHz sur la fonction / générateur de forme d'onde arbitraire, Royaume-Uni) en mode de fonctionnement sinusoïdal. Un amplificateur (Amplifier research, modèle 1W1000B, 1-1000 MHz, AR) est utilisé pour amplifier le signal provenant du générateur de fonction et obtenir l'amplitude nécessaire pour entraîner les particules. Un oscilloscope (Tektronix, modèle 1042 SCT, USA) est connecté en parallèle avec le capteur pour mesurer la tension de fonctionnement.

**B) Préparation d' échantillons alimentaires de poulet et de bœuf.**

Différentes parties de poulet (ailes, cuisses avec la peau, blancs avec et sans la peau ainsi que de la peau du cou) ont été achetées à partir de trois fournisseurs pour constituer un premier lot d'échantillons, référencés selon la partie du poulet testée. Pour chaque échantillon de ce lot, une fraction de 10 g est pesée et positionnée dans un sac TEMPO® (bioMérieux, France, réf. 80015) puis mélangé avec 90 mL d'eau peptonée tamponnée (BPW ; bioMérieux, France, réf. 42042. BPW signifiant « Buffer Peptone Water » en langue anglaise). L'ensemble est alors homogénéisé dans un système « broyeur homogénéiseur » (Masticator; IUL Instruments, Allemagne) fixé à 0,7 pour 30 s. Après homogénéisation, on procède à une étape d'enrichissement de l'échantillon par incubation pendant 4h à 37°C puis on récupère une fraction aliquote de 10 mL de bouillon enrichi prélevée du côté de la partie filtrée du sac TEMPO® (le sac TEMPO® présentant un filtre latéral permettant une préfiltration de l'échantillon). Aucune étape de filtration ultérieure n'est réalisée sur ce lot.

Un second lot d'échantillons, préparé comme précédemment est obtenu en mélangeant 10g de viande de boeuf hachée et 90 mL de BPW est réalisé de la même manière que le premier lot d'échantillon.

**C) Préparation d' échantillons inoculés**

Afin de préparer un échantillon inoculé contenant un inoculum contrôlé d'organismes pathogène, une colonie isolée de *Salmonella typhimurium* (NCTC 12023/ATCC® 14028, Pro-lab Diagnostics, UK) isolée à partir d'une culture sur milieu de culture Trypticase Soja Agar (TSA ; bioMérieux, réf. 43011, France) est mise en suspension dans 9 ml de bouillon Bouillon Trypticase Soja (TSB ; bioMérieux, réf. 42100, France) puis incubée à 37 ° C pendant 18 h. Environ 0,2 ml de bouillon enrichi ainsi obtenu est dilué dans 9 ml de BPW et son absorbance à 600 nm mesurée (appareil CECIL 1011, série 1000) pour obtenir une valeur de 0,070 correspondant à environ $1.5 \times 10^8$ UFC.mL-1. A partir de ce bouillon enrichi, 5 séries de dilutions sont ensuite réalisées et numérotées de d-1 à d-5 correspondant à des concentrations de $10^7$ à $10^3$ UFC.mL$^{-1}$, (UFC signifiant Unité Formant Colonie, ou CFU en langue anglaise pour « Colony Forming Unit »). Afin de déterminer la quantité de pathogènes présents dans le lot d'échantillon contenant un inoculum contrôlé ainsi obtenu, un milieu de culture TSA est ensemencé par 100 $\mu$l de d-5 et incubé à 37 ° C pendant une nuit. Cette étape est réalisée en doublon. Le nombre réel de microorganismes présents dans l'échantillon initial a été calculé en fonction de la dilution et du nombre de colonies comptées sur le milieu.

A partir de ces dilutions contenant des agents pathogènes, une première série de 10 ml de bouillon de poulet enrichi par incubation (préparé comme décrit ci-dessus) a été ensemencée avec 0,1 ml de $10^6$ CFU.mL$^{-1}$ *Salmonella typhimurium* pour obtenir une concentration finale de $10^4$ CFU.ml$^{-1}$.

Une seconde série de 10 ml de bouillon de poulet mélangé au BPW et préparé sans l'étape d'incubation est additionnée de 0,1 ml de $10^5$ CFU.mL$^{-1}$ de *Salmonella typhimurium* pour obtenir une concentration finale de $10^3$ CFU.mL$^{-1}$.

De la même manière, 10 ml de bouillon de bœuf à $10^3$ CFU ml$^{-1}$ sont préparés, sans l'étape d'incubation, ainsi que des échantillons à $10^4$ CFU ml$^{-1}$, avec l'étape d'incubation.

Pour la réalisation de la méthode de traitement selon l'invention, 0.5 mL d'échantillon de poulet sont introduits par deux seringues dans les orifices d'entrée 30a et 30c d'un dispositif tel que décrit précédemment à un débit de 10 $\mu$L.min$^{-1}$ par les canaux d'acheminement 40a, 40c. Un bouillon BPW est introduit par une seringue dans l'orifice d'entrée 30b à un débit de 30 $\mu$L.min$^{-1}$.

Pour la réalisation de la méthode de traitement selon l'invention , 0,5 mL d'échantillon de boeuf sont introduits par deux seringues dans les orifices d'entrée 30a et 30c d'un dispositif tel que décrit précédemment à un débit de 10$\mu$L.min$^{-1}$ par les canaux d'acheminement 40a, 40c. Un bouillon BPW est introduit par une seringue dans l'orifice d'entrée 30b à un débit de 30 $\mu$L.min$^{-1}$.

Lorsque qu'aucune signal de commande n'est pas appliqué au transducteur ultrasonique, les débris non-spécifiques de poulet ou de bœuf se retrouvent majoritairement dans les orifices de sorties 60a et 60c.

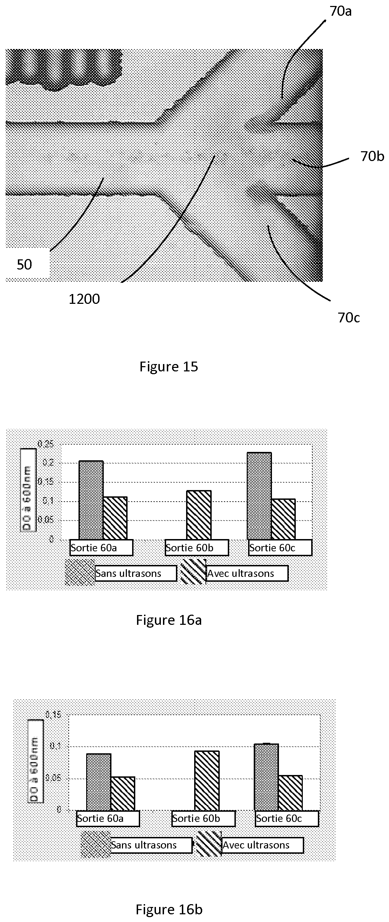

En appliquant un signal de commande au transducteur ultrasonore de 1,303 Mhz et d'amplitude de 37,4 V pic à pic (Vp-p) pour les soumettre à une étape d'acoustophorèse, et comme le montre la figure 15, un flux laminaire de débris de poulet 1200 est observé dans le canal de séparation 50 et dans le canal d'acheminement 70b en direction de l'orifice de sortie 60b. Un phénomène similaire est observable avec les débris présent dans les échantillons de bœuf. Cette observation est confirmée en mesurant la densité optique à 600nm aux différent orifices de sortie.

A partir des densités optiques (DO) mesurées, un calcul du taux de décomplexification des échantillons peut être effectué selon la relation suivante :

$$\text{Taux de décomplexification (\%)} = 100 \times \frac{\text{(DO moyenne)[sans ultrasons]} - \text{(DO moyenne)[avec ultrasons]}}{\text{(DO moyenne)[sans ultrasons]}}$$

**D) Décomplexification d' échantillons alimentaires de poulet et de bœuf.**

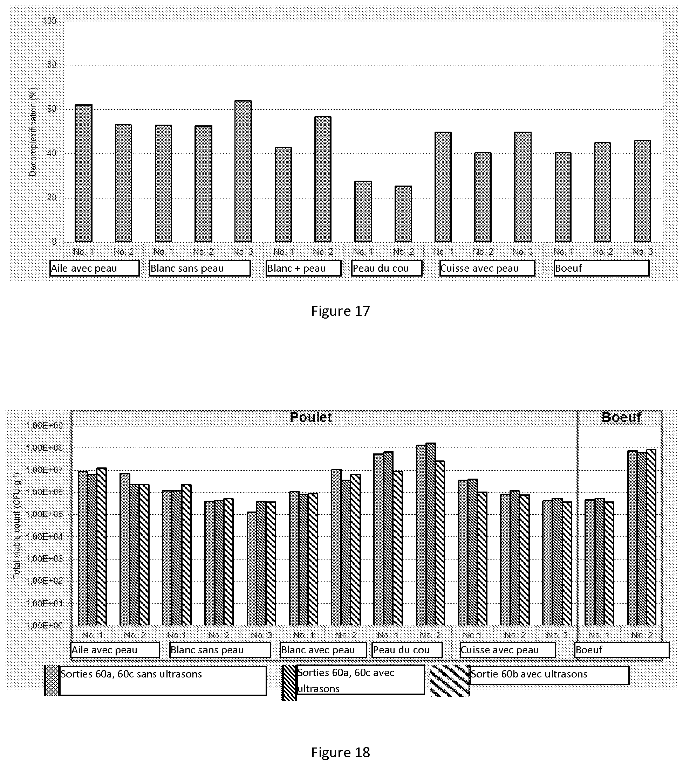

Les résultats ainsi calculés sont présentés sur la figure 16a pour le traitement des échantillons de poulet à $10^3$ CFU ml$^{-1}$ et figure 16b pour le traitement d'échantillons de bœuf à $10^3$ CFU ml$^{-1}$. Ces opérations sont reproduites deux à trois fois selon l'échantillon. Ces résultats confirment la capacité de la méthode selon l'invention à séparer et concentrer les débris dans au moins un des orifices de sorties, ici dans les orifices de sorties 60a et 60c. On note en effet sur la figure 17 un taux de décomplexification compris entre 20 et 62%.

**E) Viabilité des microorganismes présents dans des échantillons alimentaires de poulet et de bœuf.**

Après avoir démontré la capacité de décomplexification des échantillons par la méthode selon l'invention, différentes séries d'échantillon ont permis de confirmer la viabilité de la flore intrinsèque présente dans les échantillons traités par acoustophorèse selon la méthode décrite précédemment. Des tests comparatifs entre un échantillon traité avec la méthode selon l'invention et un même échantillon ayant circulé dans le dispositif sans être séparé par acoustophorèse a ainsi été effectué. A la suite de ces tests, les échantillons collectés dans les orifices de sorties du dispositif ont été collectés et ensemencés sur des géloses TSA (bioMérieux, réf. 43011, France) pour énumération de la flore totale contenue dans les échantillons après enrichissement. Quel que soit l'échantillon, poulet ou bœuf, des colonies viables ont été observées seulement dans les orifices de sorties 60a et 60c quand la séparation par acoustophorèse n'était pas activée. Ceci confirme que les débits de circulation des fluides dans le canal de séparation permettent à un flux laminaire sur toute la longueur du canal de séparation de ce mettre en place, évitant tout mélange entre les fluides. Quand la séparation par acoustophorèse est activée, des microorganismes viables ont étés retrouvés dans les échantillons provenant des trois sorties 60a, 60b, 60c, suggérant qu'une part des microorganismes ainsi que les débris présents dans l'échantillon subissent un effet de focalisation acoustique. Ces résultats sont illustrés sur la figure 18 et reproduits à deux ou trois reprises suivant les types d'échantillons. La méthode selon l'invention permet donc une préparation rapide d'échantillons alimentaires, même très complexes tel que la peau de cou de poulet, sans impact sur la flore intrinsèque et permettant des étapes de détection ultérieures.

**F) Traitement d'échantillons alimentaires de poulet et de bœuf contenant des microorganismes pathogènes**

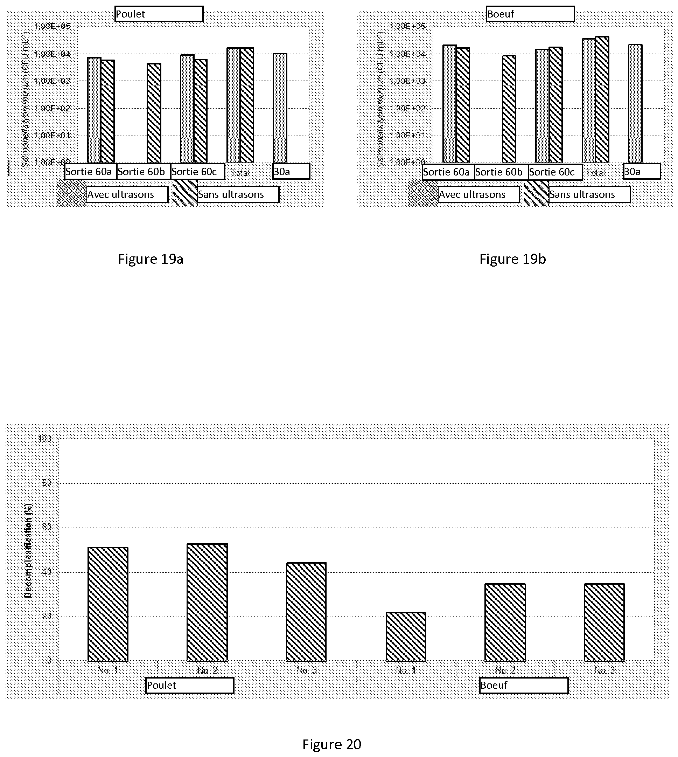

L'objectif du traitement d'échantillons alimentaires et d'être capable de séparer la flore intrinsèque des particules d'intérêts, notamment des agents pathogènes. Différentes séries d'échantillons ont permis de confirmer la viabilité des agents pathogènes présents dans les échantillons traités par acoustophorèse selon la méthode décrite précédemment. Pour cela, des colonies de *Salmonella typhimurium* ont été ajoutées postérieurement dans un échantillon de poulet enrichi au milieu BPW, tel que décrit précédemment, et incubées à 37 ° C pendant 4 h afin d'obtenir une concentration finale de $10^6$ CFU.ml$^{-1}$. De même que pour les échantillons non ensemencés, 18% et 36% de taux de décomplexification ont été observés sur ces échantillons contenant des colonies de *Salmonella typhimurium.* Le nombre total de colonies viables, obtenu par ensemencement et numération sur milieux TSA (bioMérieux, France), montre également l'aspect non-invasif de la méthode de préparation tel que décrit dans l'invention. En plus de l'observation du nombre total de colonies viables, une partie des échantillons décomplexifiés (provenant des sorties latérales) et des échantillons concentrés (provenant de la sortie centrale) ont été étalées sur un milieu chromogène chromID®-*Salmonella* (bioMérieux, réf. 43621, France) afin d'identifier la viabilité des agents pathogènes en aval de la préparation d'échantillon selon l'invention. Un niveau élevé de *Salmonella typhimurium* reprise dans les orifices de sorties a été noté avec un niveau légèrement inférieur de l'agent pathogène s'échappant par l'orifice de sortie 60b où les débris sont également plus concentrés que dans les orifices de sorties 60a et 60c, (figure 19a et figure 19b).
La démonstration d'un niveau élevé de récupération des Salmonella typhimurium ainsi que leur séparation de la flore intrinsèque par une étape de culture sur boîte sont ainsi démontrés.

**G) - Viabilité des microorganismes faiblement concentrés dans des échantillons alimentaires de poulet et de bœuf.**

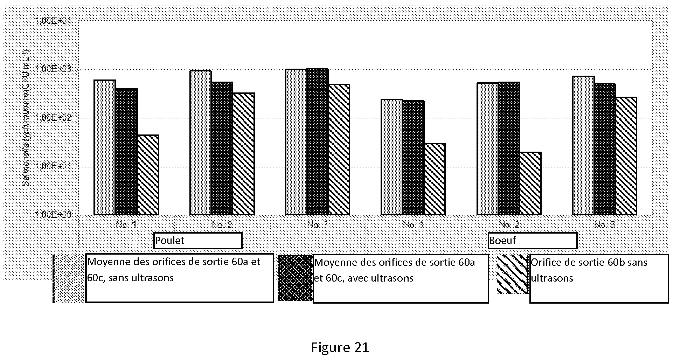

Une série d'expérience est également effectuée sur des échantillons ensemencés présentant des niveau de concentration plus faible en microorganisme pathogène cible. Pour cela, des échantillons d'une concentration en pathogène de $10^2$-$10^3$ CFU.mL$^{-1}$ sont réalisés dans l'échantillon de matrice alimentaire enrichie.
Ces échantillons ont été préparées en mélangeant 10 g d'échantillons de poulet sans la peau ou de boeuf avec 90 ml de milieu BPW, et homogénéisés par un broyeur homogénéiseur (Masticator, IUL, Allemagne) et traités sans étape d'incubation. 0,1 ml de environ $10^5$ CFU.mL$^{-1}$ de *Salmonella typhimurium* a ensuite été ensuite ensemencé dans 10 ml d'échantillon enrichi en BPW afin d'obtenir la concentration finale de $10^3$ CFU.ml$^{-1}$. Un taux de décomplexification de 20% à 50% par la méthode selon l'invention a également été observé (figure 20). De même que dans l'expérience précédente sur échantillons à forte concentration, un très haut niveau de *Salmonella typhimurium* a pu être récupéré dans les orifices de sorties 60a et 60c, selon un facteur 5 à 10 par rapport à l'orifice de sortie 60b (figure 21).

Ces exemples montrent donc l'intérêt de la méthode de traitement d'échantillon biologiques selon l'invention. Celle-ci permet donc, par exemple, à partir d'un dispositif microfluidique présentant un canal de séparation suffisamment large pour éviter que les particules non-spécifiques ne créent de blocage et associé à une onde acoustique d'une fréquence d'environ 1,3Mhz, de décomplexifier un échantillon alimentaire tout en garantissant la viabilité de la flore intrinsèque et des agents pathogènes susceptibles d'être présents, même à faible concentration en agent pathogène. Cette décomplexification s'effectue de plus à un débit rapide, d'environ 2ml.h$^{-1}$ et peut être réalisée en continue, sans nettoyage et sans risque de blocage. La résolution en microorganismes à partir des matrices alimentaires est également suffisante pour faire de la méthode selon l'invention un protocole standard, ne nécessitant pas une adaptation suivant les types d'échantillons alimentaires traités.

**Revendications**

1. Méthode de traitement d'un échantillon alimentaire susceptible de contenir une ou des espèce(s) bactériennes d'intérêt comprenant une étape de décomplexification, ladite étape de décomplexification comprenant les étapes suivantes :

   - l'introduction de tout ou partie de cet échantillon dans un premier orifice d'entrée d'un dispositif d'acoustophorèse,
   - l'introduction d'une solution tampon dans un second orifice d'entrée du dispositif d'acoustophorèse,
   - lesdits orifices d'entrée étant connectés fluidiquement à au moins deux orifices de sortie par un canal de séparation,
   - le tampon et l'échantillon étant introduits à des débits respectifs aptes à générer un flux laminaire dans le canal de séparation,
   - la réalisation d'une étape de séparation dudit échantillon par acoustophorèse de manière à favoriser la concentration des particules non-spécifiques tel que des débris alimentaires, présents dans l'échantillon dans au moins un des orifices de sortie dudit dispositif d'acoustophorèse, la fréquence d'acoustophorèse étant comprise entre 300kHz et 10 MHz, **caractérisé en ce que** le canal de séparation a :
   - une longueur comprise entre 35 mm et 80 mm
   - une forme de D dans un plan perpendiculaire à l'axe dudit canal, ledit profil comprenant une largeur inférieure Lmin au fond dudit canal comprise entre 300 $\mu$m et 357 $\mu$m, une largeur supérieure Lmax comprise entre 550 $\mu$m et 625 $\mu$m et une profondeur P comprise entre 100 $\mu$m et 150 $\mu$m.

2. Méthode de traitement selon la revendication 1 comprenant une étape de dénombrement de la flore totale présente dans l'échantillon biologique à la suite de l'étape de séparation.

3. Méthode de traitement selon la revendication 1 comprenant la réalisation d'une étape d'enrichissement de l'échantillon biologique, préférentiellement par incubation en présence d'un milieu de culture, avant l'introduction dudit échantillon biologique dans le dispositif d'acoustophorèse.

4. Méthode de traitement selon la revendication 1 ou 3 comprenant une étape de capture spécifique ou non spécifique de ou des espèce(s) d'intérêt sur un support de capture, préférentiellement sur un support de capture magnétique, suivi d'une étape de concentration par séparation immunologique ou par affinité.

5. Méthode de traitement selon l'une quelconque des revendications 3 ou 4 comprenant la réalisation d'une étape de lyse suite à l'étape de séparation ou à l'étape de capture.

6. Méthode de traitement selon la revendication 5 comprenant une étape d'amplification et d'analyse de l'échantillon lysé, préférentiellement par PCR quantitative.

7. Méthode de traitement selon l'une quelconque des revendications 3 ou 4 comprenant une étape d'analyse de ou des espèce(s) d'intérêt capturé(es), préférentiellement une étape d'identification par étalement sur un milieu de culture, alternativement par un ou des immunoessai(s) spécifique(s) de ou des espèce(s) d'intérêt capturé(es).

8. Méthode de traitement selon la revendication 3, comprenant une étape de marquage par un marqueur fluorescent spécifique de ou des espèce(s) d'intérêt suite à l'étape de séparation par acoustophorèse.

9. Méthode de traitement selon la revendication 8, comprenant une étape d'analyse en cytométrie de flux visant à détecter la présence dudit marqueur fluorescent.

10. Méthode de traitement selon l'une quelconque des revendications précédentes, l'échantillon biologique étant préalablement filtré au travers d'un filtre ou d'une membrane avant l'introduction dudit échantillon biologique dans le dispositif d'acoustophorèse.

11. Méthode de traitement selon l'une quelconque des revendications 1 à 9, l'échantillon biologique étant homogénéisé avant l'introduction dudit échantillon biologique dans le dispositif d'acoustophorèse, l'introduction se faisant sans étape préalable de filtration.

12. Méthode de traitement selon l'une quelconque des revendications précédentes comprenant la réalisation d'une seconde étape de séparation par acoustophorèse dudit échantillon alimentaire par réintroduction de l'échantillon dans le canal de séparation du dispositif ou par l'introduction de l'échantillon dans un second canal de séparation par acoustophorèse.

13. Méthode de traitement selon la revendication précédente, la seconde étape de séparation par acoustophorèse étant réalisée avec un tampon différent de la première étape.

**Patentansprüche**

1. Verfahren zur Behandlung einer Lebensmittelprobe, die eine oder mehrere interessierende bakterielle Spezies enthalten kann, welches einen Schritt der Dekomplexifikation umfasst, wobei der Schritt der Dekomplexifikation die folgenden Schritte umfasst:

   - die Einführung dieser gesamten Probe oder eines Teils davon in eine erste Einlassöffnung einer Akustophoresevorrichtung,
   - die Einführung einer Pufferlösung in eine zweite Einlassöffnung der Akustophoresevorrichtung,
   - wobei die Einlassöffnungen mit wenigstens zwei Auslassöffnungen über einen Separationskanal in Fluidverbindung stehen,
   - wobei der Puffer und die Probe mit jeweiligen Durchsätzen eingeführt werden, die geeignet sind, eine laminare Strömung in dem Separationskanal zu erzeugen,
   - die Durchführung eines Schrittes der Separation der Probe durch Akustophorese, um so die Konzentration der nichtspezifischen Partikel, wie etwa der Nahrungsreste, die in der Probe vorhanden sind, in wenigstens einer der Auslassöffnungen der Akustophoresevorrichtung zu fördern, wobei die Frequenz der Akustophorese zwischen 300 kHz und 10 MHz liegt,

   **dadurch gekennzeichnet, dass** der Separationskanal aufweist:

   - eine Länge zwischen 35 mm und 80 mm,
   - eine D-Form in einer zur Achse des Kanals senkrechten Ebene, wobei das Profil eine untere Breite Lmin am Boden des Kanals zwischen 300 $\mu$m und 357 $\mu$m, eine obere Breite Lmax zwischen 550 $\mu$m und 625 $\mu$m und eine Tiefe P zwischen 100 $\mu$m und 150 $\mu$m aufweist.

2. Verfahren zur Behandlung nach Anspruch 1, welches einen Schritt der Bestimmung der in der biologischen Probe vorhandenen Gesamtkeimzahl im Anschluss an den Schritt der Separation umfasst.

3. Verfahren zur Behandlung nach Anspruch 1, welches die Durchführung eines Schrittes der Anreicherung der biologischen Probe, vorzugsweise durch Inkubation in Gegenwart eines Kulturmediums, vor der Einführung der biologischen Probe in die Akustophoresevorrichtung umfasst.

4. Verfahren zur Behandlung nach Anspruch 1 oder 3, welches einen Schritt des spezifischen oder nichtspezifischen Einfangens des oder der interessierenden Spezies auf einem Einfang-Träger, vorzugsweise auf einem magnetischen Einfang-Träger, umfasst, gefolgt von einem Schritt der Konzentration durch immunologische Separation oder durch Affinität.

5. Verfahren zur Behandlung nach einem der Ansprüche 3 oder 4, welches die Durchführung eines Schrittes der Lyse im Anschluss an den Schritt der Separation oder an den Schritt des Einfangens umfasst.

6. Verfahren zur Behandlung nach Anspruch 5, welches einen Schritt der Amplifikation und der Analyse der lysierten Probe umfasst, vorzugsweise durch quantitative PCR.

**7.** Verfahren zur Behandlung nach einem der Ansprüche 3 oder 4, welches einen Schritt der Analyse der einen oder mehreren eingefangenen interessierenden Spezies umfasst, vorzugsweise einen Schritt der Identifizierung durch Ausstreichen auf einem Kulturmedium, alternativ dazu durch einen oder mehrere spezifische Immunassays der einen oder mehreren eingefangenen interessierenden Spezies.

**8.** Verfahren zur Behandlung nach Anspruch 3, welches einen Schritt der Markierung der einen oder mehreren interessierenden Spezies mit einem spezifischen Fluoreszenzmarker im Anschluss an den Schritt der Separation durch Akustophorese umfasst.

**9.** Verfahren zur Behandlung nach Anspruch 8, welches einen Schritt der Analyse mittels Strömungszytometrie zum Erkennen des Vorhandenseins des Fluoreszenzmarkers umfasst.

**10.** Verfahren zur Behandlung nach einem der vorhergehenden Ansprüche, wobei die biologische Probe vor der Einführung der biologischen Probe in die Akustophoresevorrichtung zuvor durch ein Filter oder eine Membran gefiltert wird.

**11.** Verfahren zur Behandlung nach einem der Ansprüche 1 bis 9, wobei die biologische Probe vor der Einführung der biologischen Probe in die Akustophoresevorrichtung homogenisiert wird, wobei die Einführung ohne vorausgehenden Schritt der Filtration erfolgt.

**12.** Verfahren zur Behandlung nach einem der vorhergehenden Ansprüche, welches die Durchführung eines zweiten Schrittes der Separation der Lebensmittelprobe durch Akustophorese durch erneute Einführung der Probe in den Separationskanal der Vorrichtung oder durch die Einführung der Probe in einen zweiten Kanal zur Separation durch Akustophorese umfasst.

**13.** Verfahren zur Behandlung nach dem vorhergehenden Anspruch, wobei der zweite Schritt der Separation durch Akustophorese mit einem anderen Puffer als der erste Schritt durchgeführt wird.

**Claims**

**1.** Method for treatment of a food sample which may contain one or more bacterial species of interest, comprising a decomplexification step, said decomplexification step comprising the following steps :

- introducing all or part of this sample into a first inlet orifice of an acoustophoresis device,
- introducing a buffer solution into a second inlet orifice of the acoustophoresis device,
- said inlet orifices being fluidically connected to at least two outlet orifices by a separation channel,
- the buffer and the sample being introduced at respective flow rates capable of generating a laminar flow in the separation channel,
- carrying out a step of separation of said sample by acoustophoresis so as to promote the concentration of the non-specific particles, such as food debris, present in the sample in at least one of the outlet orifices of said acoustophoresis device, the frequency of acoustophoresis being between 300 kHz and 10MHz,

**characterized in that** the separation channel comprises :

- a length between 35 mm and 80mm,
- a "D" shape in a perpendicular plane regarding the channel's axis, the cross-section comprising an inferior width Lmin at the bottom of the channel between 300 $\mu$m and 357 $\mu$m, a superior width Lmax between 550 $\mu$m and 625 $\mu$m, and a depth P between 100 $\mu$m and 150 $\mu$m.

**2.** Treatment method according to claim 1, comprising a step of counting the total flora present in the biological sample following the separation step.

**3.** Treatment method according to claim 1, comprising carrying out a step of enrichment of the biological sample, preferentially by incubation in the presence of a culture medium, before the introduction of said biological sample into the acoustophoresis device.

**4.** Treatment method according to claim 1 or 3, comprising a step of specific or non-specific capture of the one or more species of interest on a capture support, preferentially on a magnetic capture support, followed by a step of concentration by immunological separation or by affinity separation.

5. Treatment method according to any one of the claims 3 or 4, comprising carrying out a lysis step following the separation step or following the capture step.

6. Treatment method according to claim 5, comprising a step of amplification and analysis of the lysed sample, preferentially by quantitative PCR.

7. Treatment method according to any one of the claims 3 or 4, comprising a step of analysis of the one or more species of interest captured, preferentially a step of identification by plating out on a culture medium, alternatively by means of one or more immunoassay(s) specific for the one or more species of interest captured.

8. Treatment method according to claim 3, comprising a step of labeling with a fluorescent label specific for the one or more species of interest following the step of separation by acoustophoresis.

9. Treatment method according to claim 8, comprising a step of analysis by flow cytometry aimed at detecting the presence of said fluorescent label.

10. Treatment method according to any one of the preceding claims, the biological sample being previously filtered through a filter or a membrane before the introduction of said biological sample into the acoustophoresis device.

11. Treatment method according to any one of claims 1 to 9, the biological sample being homogenized before the introduction of said biological sample into the acoustophoresis device, the introduction being carried out without prior filtration step.

12. Treatment method according to any one of the preceding claims, comprising carrying out a second step of separation by acoustophoresis of said food sample by reintroduction of the sample into the separation channel of the device or by introduction of the sample into a second channel of separation by acoustophoresis.

13. Treatment method according to the preceding claim, the second step of separation by acoustophoresis being carried out with a buffer different than the first step.

Figure 1

Coupe A-A

Figure 2a

Lmax

P

50

Lmin

Coupe B-B

Figure 2b

Oscilloscope

120

10

110

130a

Echantillon
biologique

130b

Tampon / Milieu

Echantillon
biologique

130c

Générateur
d'ondes

140a

Echantillon
décomplexifié

140b

Particules non
spécifiques

Echantillon
décomplexifié

140c

90

Détail A

Figure 3

Figure 4

Figure 5a – Coupe B-B

Figure 5b

Figure 6a

Figure 6b

Figure 6c

1400

1470a

1440a

1430a 1430b 1422 1470b 1440a' 1470f 1470d

1440b 1450 1430b' 1450' 1470e 1460c 1460a
1440c 1440b' 1460c 1460b
1470c 1440c'

Figure 6d

370a 2000 300

M

N S N

S N S

M

2100 2000

360c 360b 2000 360a

3100 300

370a Raman ID

360c 360b 3000 360a

Figure 7a                    Figure 7b

33

500

530a

530b

560b

560a

521

540c

570c

540a

540b    590b    590a    550    570b    570a

522

Figure 8

690    500

560a

610

560b

600

Figure 9

Figure 10

Figure 11

Figure 12a - Coupe A-A

Figure 12b - Coupe B-B

Figure 13a

Figure 13b – Coupe A-A

Figure 14

70a

70b

70c

50

1200

Figure 15

Figure 16a

Figure 16b

Figure 17

Figure 18

Figure 19a

Figure 19b

Figure 20

Figure 21

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **PERAUGUSTSSON et al.** Decomplexing Biofluids using microchip based acoustophoresis. *LAB ON CHIP,* 2009, vol. 9 (6), 801-818

- **L.C. JELLEMA et al.** Acoustofluidics 8 : Applications of acoustophoresis in continuous flow microsvstems. *LAB ON CHIP,* 2012, vol. 12 (7), 1210-1223